# EUROPEAN PATENT APPLICATION

(11) **EP 3 747 471 A1**
(43) Date of publication of application: **09.12.2020**
(21) Application number: 19747229.3
(22) Date of filing: 01.02.2019
(51) Int. Cl.: A61K 45/00, A61K 31/135, A61K 31/397, A61K 31/403, A61K 31/4245, A61K 31/426, A61K 31/496, A61P 9/10, A61P 27/02

(54) **MEDICINE FOR PREVENTING OR TREATING OPHTHALMIC DISEASE ASSOCIATED WITH ENHANCED INTRAOCULAR NEOVASCULARIZATION AND/OR INTRAOCULAR VASCULAR PERMEABILITY**

(30) Priority: 02.02.2018 JP 2018016911
(71) Applicant: Kyoto University, Kyoto-shi, Kyoto 606-8501 (JP); Astellas Pharma Inc., Chuo-ku Tokyo 103-8411 (JP)
(72) Inventor: HARA, Hideaki, Gifu-shi, Gifu 501-1196 (JP); NARUMIYA, Shuh, Kyoto-shi, Kyoto 606-8501 (JP); AOKI, Tomohiro, Kyoto-shi, Kyoto 606-8501 (JP); ARAMORI, Ichiro, Tokyo 103-8411 (JP); YAMAMOTO, Rie, Tokyo 103-8411 (JP)
(74) Representative: Hoffmann Eitle
(86) International application number: PCT/JP2019/003573
(87) International publication number: WO 2019/151470

(57) **Abstract**

Provided is a pharmaceutical for preventing or treating an ophthalmic disease associated with intraocular neovascularization and/or increased intraocular vascular permeability. The inventors of the present invention have made investigations on a pharmaceutical for preventing or treating an ophthalmic disease associated with intraocular neovascularization and/or increased intraocular vascular permeability, and have confirmed that a selective S1P receptor agonist having agonist activity at an S1P₁ receptor has an intraocular neovascularization-reducing action and an intraocular vascular permeability-reducing action, thus completing the present invention. A compound or a pharmaceutically acceptable salt thereof of the present invention, which serves as the selective S1P receptor agonist having agonist activity at the S1P₁ receptor, has an intraocular neovascularization-reducing action and an intraocular vascular permeability-reducing action, and can be used as a preventive and/or therapeutic agent for, for example, exudative age-related macular degeneration, diabetic retinopathy, diabetic macular edema, myopic choroidal neovascularization, retinal artery occlusion, retinal vein occlusion, or neovascular glaucoma.

## Description

### Technical Field

The present invention relates to a pharmaceutical for preventing or treating an ophthalmic disease associated with intraocular neovascularization and/or increased intraocular vascular permeability.

The present application claims priority from Japanese Patent Application No. 2018-016911, which is incorporated herein by reference.

### Background Art

There are known various ophthalmic diseases each being associated with intraocular neovascularization or increased intraocular vascular permeability. For example, age-related macular degeneration is a degenerative disease of the retinal macula associated with aging, and is a refractory ophthalmic disease associated with a severe lowering of eyesight. The age-related macular degeneration is classified into an "exudative type", which is associated with undesirable choroidal neovascularization and causes a rapid lowering of eyesight, and an "atrophic type", which is not associated with choroidal neovascularization, and in which atrophy of photoreceptor cells gradually progresses to cause lowering of eyesight. New blood vessels in the exudative age-related macular degeneration are fragile and easily broken, and hence bleeding or leakage of water in blood (exudate) occurs to accumulate in a tissue. Accordingly, edema occurs to impair the function of the retina or the macula. In addition, in each of diabetic retinopathy and diabetic macular edema, a hyperglycemic state continues for a long period of time, resulting in exudation of blood components from blood vessels in the retina, or resulting in formation of undesirable new blood vessels and bleeding from the new blood vessels or leakage of water in blood (exudate) therefrom. Accordingly, fundus bleeding, retinal edema, or the like occurs to impair the function of the retina or the macula.

Hitherto, there have been proposed therapeutic agents targeting vascular endothelial growth factor (hereinafter referred to as "VEGF") because VEGF plays important roles in development of choroidal neovascularization and increase of vascular permeability. Examples thereof include an anti-VEGF antibody (WO 2008/063932A2), and a combination of ananti-VEGFagent and a specific low-molecular-weight compound (WO 2011/087066 A1 and WO 2012/105610 A1). Besides, for the age-related macular degeneration, treatment using a VEGF inhibitor, such as pegaptanib sodium (product name: Macugen), ranibizumab (product name: Lucentis), or aflibercept (product name: Eylea), is clinically performed. However, the VEGF inhibitor used for the treatment is used as an intravitreal injection, and hence puts huge psychological and physical burdens on a patient or clinical side when administered many times.

In addition, there is a report that a timing of administration is important when the anti-VEGF antibody is to be used because, when the anti-VEGF antibody was administered immediately after occlusion of a retinal vein in a retinal vein occlusion (RVO) model, its non perfusion area was decreased, whereas when the anti-VEGF antibody was administered 7 days after the occlusion, the non perfusion area was enlarged (Scientific Reports, 2017, vol. 7, p. 43509).

Meanwhile, with regard to an ophthalmic disease associated with an increase in level of sphingosine-1-phosphate (hereinafter referred to as "S1P"), such as age-related macular degeneration, choroidal neovascularization, retinopathy of prematurity, or retinopathy, there is a proposal of a method involving inhibiting binding of ligand molecules to S1P₁ to S1P₅ receptors through use of anti-SIP antibodies, to thereby reduce abnormal neovascularization and the like (WO 2008/055072A2 and WO 2007/053447 A2). However, the use of the antibodies as active ingredients limits use of the method to an intraocular injection.

In addition, there is a proposal that an ophthalmic disease due to vascular permeability disorder, such as diabetic retinopathy, be treated with a low-molecular-weight compound having agonist activity at an S1P receptor. For example, there is a proposal that an S1P receptor agonist having agonist activity at one or a plurality of receptors out of the S1P₁ receptor to the S1P₅ receptor, in particular, FTY720 (Fingolimod) be used as a medicament for treating vascular permeability disorders and unwanted vascular endothelial cell apoptosis, and for the growth of new blood vessels (Patent Literature 1).

In addition, there are proposals of many novel low-molecular-weight compounds each having agonist activity at the S1P₁ receptor (Patent Literature 2 and Patent Literature 3). For example, there are reports of: compounds each having an inhibitory action on binding between S1P and the S1P₁ receptor (WO 2006/013948 A1, WO 2007/089018 A1, WO 2007/091570 A1, and WO 2009/017219 A1); compounds which signal as agonists at the S1P receptors, i.e., the S1P₁ receptor to the S1P₅ receptor (WO 2006/094705 A1); and compounds each having agonist activity at the S1P₁ receptor, for decreasing circulating lymphocytes in blood (WO 2008/152149 A1, WO 2012/145236 A1, WO 2011/066184 A1, WO 2012/071186 A1, WO 2012/071184 A1, WO 2011/050054 A2, WO 2011/066179 A1, WO 2012/074921 A1, US 2011/0257232 A1, WO 2012/142268 A1, WO 2014/130565 A1, WO 2014/078209 A1, WO 2014/078208 A1, and WO 2014/071342 A1). Besides, many diseases are mentioned as examples of applications of the compounds, but there is no specific description of results that have been obtained by using a compound having S1P receptor agonist activity or a pharmaceutically acceptable salt thereof, and that confirm its effect on an ophthalmic disease associated with intraocular neovascularization or increased intraocular vascular permeability, such as age-related maculopathy or diabetic retinopathy.

In addition, there is no report proposing use of an S1P receptor agonist for an ophthalmic disease with attention focused on its barrier function-enhancing action between vascular endothelial cells. Some of the inventors of the present invention previously proposed an invention in which an S1P receptor agonist was used for treatment of cerebral aneurysm (Patent Literature 4) . It was shown that the treatment was achieved because, as its action, the S1P receptor agonist reduced permeability of vascular endothelial cells to decrease the number of macrophages infiltrating cerebral aneurysm walls. However, there is no description that the S1P receptor agonist can be applied to an ophthalmic disease.

### Citation List

### Patent Literature

[PTL 1] WO 2005/002559 A2
[PTL 2] WO 2007/116866 A1
[PTL 3] WO 2010/064707 A1
[PTL 4] WO 2014/175287 A1

### Summary of Invention

### Technical Problem

There is provided a pharmaceutical useful for preventing or treating an ophthalmic disease associated with intraocular neovascularization and/or increased intraocular vascular permeability.

### Solution to Problem

The inventors of the present invention have made investigations as to whether an S1P receptor agonist can be used for preventing or treating an ophthalmic disease associated with intraocular neovascularization and/or increased intraocular vascular permeability, with attention focused particularly on enhancing a barrier function between vascular endothelial cells, and as a result, have found that the desired object is achieved by a compound having a specific S1P receptor agonist activity profile.

That is, the inventors have made extensive investigations on a selective S1P receptor agonist having agonist activity at an S1P₁ receptor, and as a result, have found that the selective S1P receptor agonist is effective for an ophthalmic disease associated with intraocular neovascularization and/or increased intraocular vascular permeability. Thus, the inventors have completed the present invention.

The present invention relates to a pharmaceutical for preventing or treating an ophthalmic disease associated with intraocular neovascularization and/or increased intraocular vascular permeability, the pharmaceutical including as an active ingredient a selective S1P receptor agonist, that is, a compound that has agonist activity at an S1P₁ receptor, and that may further have agonist activity at one or both of an S1P₄ receptor and an S1P₅ receptor, or a pharmaceutically acceptable salt thereof.

The present invention also relates to a pharmaceutical composition for preventing or treating an ophthalmic disease associated with intraocular neovascularization and/or increased intraocular vascular permeability, the pharmaceutical composition including a selective S1P receptor agonist and a pharmaceutically acceptable excipient. The pharmaceutical composition encompasses a preventive or therapeutic agent for an ophthalmic disease associated with intraocular neovascularization and/or increased intraocular vascular permeability, the preventive or therapeutic agent including a selective S1P receptor agonist.

The present invention also relates to: a use of a selective S1P receptor agonist for manufacture of a pharmaceutical composition for preventing or treating an ophthalmic disease associated with intraocular neovascularization and/or increased intraocular vascular permeability; a use of a selective S1P receptor agonist for preventing or treating an ophthalmic disease associated with intraocular neovascularization and/or increased intraocular vascular permeability; a selective S1P receptor agonist for use in prevention or treatment of an ophthalmic disease associated with intraocular neovascularization and/or increased intraocular vascular permeability; and a method of preventing or treating an ophthalmic disease associated with intraocular neovascularization and/or increased intraocular vascular permeability, the method including administering an effective dose of a selective S1P receptor agonist to a subject. The term "subject" refers to a human or any other animal in need of the prevention or treatment, and in one embodiment, refers to a human in need of the prevention or treatment.

That is, the present invention includes the following items.
(1) A pharmaceutical composition for preventing or treating an ophthalmic disease associated with intraocular neovascularization and/or increased intraocular vascular permeability, the pharmaceutical composition including as an active ingredient a selective S1P receptor agonist having agonist activity at an S1P₁ receptor.
(2) The pharmaceutical composition according to Item (1), wherein the selective S1P receptor agonist is a compound having S1P₁ receptor agonist activity and further having agonist activity at one or both of an S1P₅ receptor and an S1P₄ receptor, or a pharmaceutically acceptable salt thereof.
(3) The pharmaceutical composition according to Item (1), wherein the selective S1P receptor agonist is a compound having S1P₁ receptor agonist activity and further having agonist activity at an S1P₅ receptor, or a pharmaceutically acceptable salt thereof.
(4) The pharmaceutical composition according to any one of Items (1) to (3), wherein the selective S1P receptor agonist is a compound or a pharmaceutically acceptable salt thereof selected from the group consisting of: 5-{5-[3-(trifluoromethyl)-4-{[(2S)-1,1,1-trifluoropropan-2-yl] oxy}phenyl]-1,2,4-oxadiazol-3-yl}-1H-benzimidazole or a pharmaceutically acceptable salt thereof; 1-[(7-{[4-(2,2,2-trifluoroethoxy)-3-(trifluoromethyl)phenyl]me thoxy}-2H-1-benzopyran-3-yl)methyl]piperidine-4-carboxylic acid or a pharmaceutically acceptable salt thereof; 1-({4-[(1E)-N-{[4-cyclohexyl-3-(trifluoromethyl)phenyl]methoxy }ethanimidoyl]-2-ethylphenyl}methyl)azetidine-3-carboxylic acid or a pharmaceutically acceptable salt thereof; 5-(3-{(1S)-1-[(2-hydroxyethyl)amino]-2,3-dihydro-1H-inden-4-yl }-1,2,4-oxadiazol-5-yl)-2-[(propan-2-yl)oxy]benzonitrile or a pharmaceutically acceptable salt thereof; (2Z,5Z)-5-({3-chloro-4-[(2R)-2,3-dihydroxypropoxy]phenyl}methy lidene)-3-(2-methylphenyl)-2-(propylimino)-1,3-thiazolidin-4-o ne or a pharmaceutically acceptable salt thereof; [(3R)-7-{[4-cyclopentyl-3-(trifluoromethyl)phenyl]methoxy}-1,2 ,3,4-tetrahydrocyclopenta[b]indol-3-yl]acetic acid or a pharmaceutically acceptable salt thereof; and 2-amino-2-[2-(4-{[3-(benzyloxy)phenyl]sulfanyl}-2-chlorophenyl )ethyl]propane-1,3-diol or a pharmaceutically acceptable salt thereof.
(5) The pharmaceutical composition according to Item (4), wherein the selective S1P receptor agonist is 5-{5-[3-(trifluoromethyl)-4-{[(2S)-1,1,1-trifluoropropan-2-yl] oxy}phenyl]-1,2,4-oxadiazol-3-yl}-1H-benzimidazole or a pharmaceutically acceptable salt thereof.
(6) The pharmaceutical composition according to any one of Items (1) to (5), wherein the ophthalmic disease associated with intraocular neovascularization and/or increased intraocular vascular permeability is exudative age-related macular degeneration, diabetic retinopathy, diabetic macular edema, retinopathy of prematurity, myopic choroidal neovascularization, secondary choroidal neovascularization, retinal artery occlusion, retinal vein occlusion, neovascularglaucoma, retinitispigmentosa, or edema caused by retinal photocoagulation.
(7) The pharmaceutical composition according to any one of Items (1) to (5), wherein the ophthalmic disease associated with intraocular neovascularization and/or increased intraocular vascular permeability is exudative age-related macular degeneration, diabetic retinopathy, diabetic macular edema, myopic choroidal neovascularization, retinal artery occlusion, retinal vein occlusion, or neovascular glaucoma.
(8) The pharmaceutical composition according to any one of Items (1) to (7), wherein the pharmaceutical composition is intraocularly injected or intravitreally injected to treat the ophthalmic disease associated with intraocular neovascularization and/or increased intraocular vascular permeability.
(9) The pharmaceutical composition according to Item (8), wherein the pharmaceutical composition is intravitreally injected to treat the ophthalmic disease associated with intraocular neovascularization and/or increased intraocular vascular permeability.
(10) A method of preventing or treating an ophthalmic disease associated with intraocular neovascularization and/or increased intraocular vascular permeability, the method including administering an effective dose of a selective S1P receptor agonist having agonist activity at an S1P₁ receptor to a subject.
(11) The method according to Item (10), wherein the selective S1P receptor agonist is a compound having S1P₁ receptor agonist activity and further having agonist activity at one or both of an S1P₅ receptor and an S1P₄ receptor, or a pharmaceutically acceptable salt thereof.
(12) The method according to Item (10), wherein the selective S1P receptor agonist is a compound having S1P₁ receptor agonist activity and further having agonist activity at an S1P₅ receptor, or a pharmaceutically acceptable salt thereof.
(13) The method according to any one of Items (10) to (12), wherein the selective S1P receptor agonist is a compound or a pharmaceutically acceptable salt thereof selected from the group consisting of: 5-{5-[3-(trifluoromethyl)-4-{[(2S)-1,1,1-trifluoropropan-2-yl] oxy}phenyl]-1,2,4-oxadiazol-3-yl}-1H-benzimidazole or a pharmaceutically acceptable salt thereof; 1-[(7-{[4-(2,2,2-trifluoroethoxy)-3-(trifluoromethyl)phenyl]me thoxy}-2H-1-benzopyran-3-yl)methyl]piperidine-4-carboxylic acid or a pharmaceutically acceptable salt thereof; 1-({4-[(1E)-N-{[4-cyclohexyl-3-(trifluoromethyl)phenyl]methoxy }ethanimidoyl]-2-ethylphenyl}methyl)azetidine-3-carboxylic acid or a pharmaceutically acceptable salt thereof; 5-(3-{(1S)-1-[(2-hydroxyethyl)amino]-2,3-dihydro-1H-inden-4-yl }-1,2,4-oxadiazol-5-yl)-2-[(propan-2-yl)oxy]benzonitrile or a pharmaceutically acceptable salt thereof; (2Z,5Z)-5-({3-chloro-4-[(2R)-2,3-dihydroxypropoxy]phenyl}methy lidene)-3-(2-methylphenyl)-2-(propylimino)-1,3-thiazolidin-4-o ne or a pharmaceutically acceptable salt thereof; [(3R)-7-{ [4-cyclopentyl-3-(trifluoromethyl)phenyl]methoxy}-1,2 ,3,4-tetrahydrocyclopenta[b]indol-3-yl]acetic acid or a pharmaceutically acceptable salt thereof; and 2-amino-2-[2-(4-{[3-(benzyloxy)phenyl]sulfanyl}-2-chlorophenyl )ethyl]propane-1,3-diol or a pharmaceutically acceptable salt thereof.
(14) The method according to Item (13), wherein the selective S1P receptor agonist is 5-{5-[3-(trifluoromethyl)-4-{[(2S)-1,1,1-trifluoropropan-2-yl] oxy}phenyl]-1,2,4-oxadiazol-3-yl}-1H-benzimidazole or a pharmaceutically acceptable salt thereof.
(15) The method according to any one of Items (10) to (14), wherein the ophthalmic disease associated with intraocular neovascularization and/or increased intraocular vascular permeability is exudative age-related macular degeneration, diabetic retinopathy, diabetic macular edema, retinopathy of prematurity, myopic choroidal neovascularization, secondary choroidal neovascularization, retinal artery occlusion, retinal vein occlusion, neovascularglaucoma, retinitis pigmentosa, or edema caused by retinal photocoagulation.
(16) The method according to any one of Items (10) to (14), wherein the ophthalmic disease associated with intraocular neovascularization and/or increased intraocular vascular permeability is exudative age-related macular degeneration, diabetic retinopathy, diabetic macular edema, myopic choroidal neovascularization, retinal artery occlusion, retinal vein occlusion, or neovascular glaucoma.
(17) The method according to any one of Items (10) to (16), wherein a method for the administering is intraocular injection or intravitreal injection.
(18) The method according to Item (17), wherein the method for the administering is intravitreal injection.
(19) A selective S1P receptor agonist having agonist activity at an S1P₁ receptor, for use in prevention or treatment of an ophthalmic disease associated with intraocular neovascularization and/or increased intraocular vascular permeability.
(20) The selective S1P receptor agonist according to Item (19), wherein the selective S1P receptor agonist is a compound having S1P₁ receptor agonist activity and further having agonist activity at one or both of an S1P₅ receptor and an S1P₄ receptor, or a pharmaceutically acceptable salt thereof.
(21) The selective S1P receptor agonist according to Item (19), wherein the selective S1P receptor agonist is a compound having S1P₁ receptor agonist activity and further having agonist activity at an S1P₅ receptor, or a pharmaceutically acceptable salt thereof.
(22) The selective S1P receptor agonist according to any one of Items (19) to (21), wherein the selective S1P receptor agonist is a compound or a pharmaceutically acceptable salt thereof selected from the group consisting of: 5-{5-[3-(trifluoromethyl)-4-{[(2S)-1,1,1-trifluoropropan-2-yl] oxy}phenyl]-1,2,4-oxadiazol-3-yl}-1H-benzimidazole or a pharmaceutically acceptable salt thereof; 1-[(7-{[4-(2,2,2-trifluoroethoxy)-3-(trifluoromethyl)phenyl]me thoxy}-2H-1-benzopyran-3-yl)methyl]piperidine-4-carboxylic acid or a pharmaceutically acceptable salt thereof; 1-({4-[(1E)-N-{[4-cyclohexyl-3-(trifluoromethyl)phenyl]methoxy }ethanimidoyl]-2-ethylphenyl}methyl)azetidine-3-carboxylic acid or a pharmaceutically acceptable salt thereof; 5-(3-{(1S)-1-[(2-hydroxyethyl)amino]-2,3-dihydro-1H-inden-4-yl }-1,2,4-oxadiazol-5-yl)-2-[(propan-2-yl)oxy]benzonitrile or a pharmaceutically acceptable salt thereof; (2Z,5Z)-5-({3-chloro-4-[(2R)-2,3-dihydroxypropoxy]phenyl}methy lidene)-3-(2-methylphenyl)-2-(propylimino)-1,3-thiazolidin-4-o ne or a pharmaceutically acceptable salt thereof; [(3R)-7-{ [4-cyclopentyl-3-(trifluoromethyl)phenyl]methoxy}-1,2 ,3,4-tetrahydrocyclopenta[b]indol-3-yl]acetic acid or a pharmaceutically acceptable salt thereof; and 2-amino-2-[2-(4-{[3-(benzyloxy)phenyl]sulfanyl}-2-chlorophenyl )ethyl]propane-1,3-diol or a pharmaceutically acceptable salt thereof.
(23) The selective S1P receptor agonist according to Item (22), wherein the selective S1P receptor agonist is 5-{5-[3-(trifluoromethyl)-4-{[(2S)-1,1,1-trifluoropropan-2-yl] oxy}phenyl]-1,2,4-oxadiazol-3-yl}-1H-benzimidazole or a pharmaceutically acceptable salt thereof.
(24) The selective S1P receptor agonist according to any one of Items (19) to (23), wherein the ophthalmic disease associated with intraocular neovascularization and/or increased intraocular vascular permeability is exudative age-related macular degeneration, diabetic retinopathy, diabetic macular edema, retinopathy of prematurity, myopic choroidal neovascularization, secondary choroidal neovascularization, retinal artery occlusion, retinal vein occlusion, neovascularglaucoma, retinitispigmentosa, or edema caused by retinal photocoagulation.
(25) The selective S1P receptor agonist according to any one of Items (19) to (23), wherein the ophthalmic disease associated with intraocular neovascularization and/or increased intraocular vascular permeability is exudative age-related macular degeneration, diabetic retinopathy, diabetic macular edema, myopic choroidal neovascularization, retinal artery occlusion, retinal vein occlusion, or neovascular glaucoma.
(26) The selective S1P receptor agonist according to any one of Items (19) to (25), wherein the selective S1P receptor agonist is for use in intraocular injection or intravitreal injection.
(27) The selective S1P receptor agonist according to Item (26), wherein the selective S1P receptor agonist is for use in intravitreal injection.
(28) A use of a selective S1P receptor agonist for manufacture of a pharmaceutical composition for preventing or treating an ophthalmic disease associated with intraocular neovascularization and/or increased intraocular vascular permeability, the pharmaceutical composition including as an active ingredient a selective S1P receptor agonist having agonist activity at an S1P₁ receptor.
(29) The use according to Item (28), wherein the ophthalmic disease associated with intraocular neovascularization and/or increased intraocular vascular permeability is exudative age-related macular degeneration, diabetic retinopathy, diabetic macular edema, retinopathy of prematurity, myopic choroidal neovascularization, secondary choroidal neovascularization, retinal artery occlusion, retinal vein occlusion, neovascular glaucoma, retinitis pigmentosa, or edema caused by retinal photocoagulation.
(30) The use according to Item (29), wherein the ophthalmic disease associated with intraocular neovascularization and/or increased intraocular vascular permeability is exudative age-related macular degeneration, diabetic retinopathy, diabetic macular edema, myopic choroidal neovascularization, retinal artery occlusion, retinal vein occlusion, or neovascular glaucoma.
(31) The use according to Item (30), wherein the selective S1P receptor agonist is a compound or a pharmaceutically acceptable salt thereof selected from the group consisting of: 5-{5-[3-(trifluoromethyl)-4-{[(2S)-1,1,1-trifluoropropan-2-yl] oxy}phenyl]-1,2,4-oxadiazol-3-yl}-1H-benzimidazole or a pharmaceutically acceptable salt thereof; 1-[(7-{[4-(2,2,2-trifluoroethoxy)-3-(trifluoromethyl)phenyl]me thoxy}-2H-1-benzopyran-3-yl)methyl]piperidine-4-carboxylic acid or a pharmaceutically acceptable salt thereof; 1-({4-[(1E)-N-{[4-cyclohexyl-3-(trifluoromethyl)phenyl]methoxy }ethanimidoyl]-2-ethylphenyl}methyl)azetidine-3-carboxylic acid or a pharmaceutically acceptable salt thereof; 5-(3-{(1S)-1-[(2-hydroxyethyl)amino]-2,3-dihydro-1H-inden-4-yl }-1,2,4-oxadiazol-5-yl)-2-[(propan-2-yl)oxy]benzonitrile or a pharmaceutically acceptable salt thereof; (2Z,5Z)-5-({3-chloro-4-[(2R)-2,3-dihydroxypropoxy]phenyl}methy lidene)-3-(2-methylphenyl)-2-(propylimino)-1,3-thiazolidin-4-o ne or a pharmaceutically acceptable salt thereof; [(3R)-7-{ [4-cyclopentyl-3-(trifluoromethyl)phenyl]methoxy}-1,2 ,3,4-tetrahydrocyclopenta[b]indol-3-yl]acetic acid or a pharmaceutically acceptable salt thereof; and 2-amino-2-[2-(4-{[3-(benzyloxy)phenyl]sulfanyl}-2-chlorophenyl )ethyl]propane-1,3-diol or a pharmaceutically acceptable salt thereof.
(32) The use according to Item (31), wherein the selective S1P receptor agonist is 5-{5-[3-(trifluoromethyl)-4-{[(2S)-1,1,1-trifluoropropan-2-yl] oxy}phenyl]-1,2,4-oxadiazol-3-yl}-1H-benzimidazole or a pharmaceutically acceptable salt thereof.
(33) The use according to Item (31) or (32), wherein the selective S1P receptor agonist is for use in intraocular injection or intravitreal injection.
(34) The use according to Item (33), wherein the selective S1P receptor agonist is for use in intravitreal injection.
   (A) A pharmaceutical composition for preventing or treating exudative age-related macular degeneration, diabetic retinopathy, diabetic macular edema, retinopathy of prematurity, myopic choroidal neovascularization, secondary choroidal neovascularization, retinal artery occlusion, retinal vein occlusion, neovascular glaucoma, retinitis pigmentosa, or edema caused by retinal photocoagulation, the pharmaceutical composition including as an active ingredient 5-{5-[3-(trifluoromethyl)-4-{[(2S)-1,1,1-trifluoropropan-2-yl] oxy}phenyl]-1,2,4-oxadiazol-3-yl}-1H-benzimidazole or a pharmaceutically acceptable salt thereof.
   (B) The pharmaceutical composition according to Item (A), wherein the pharmaceutical composition is intravitreally injected.
   (C) Amethod of preventing or treating exudative age-related macular degeneration, diabetic retinopathy, diabetic macular edema, retinopathy of prematurity, myopic choroidal neovascularization, secondary choroidal neovascularization, retinal artery occlusion, retinal vein occlusion, neovascular glaucoma, retinitispigmentosa, or edema caused by retinal photocoagulation, the method including administering an effective dose of 5-{5-[3-(trifluoromethyl)-4-{[(2S)-1,1,1-trifluoropropan-2-yl] oxy}phenyl]-1,2,4-oxadiazol-3-yl}-1H-benzimidazole or a pharmaceutically acceptable salt thereof to a subject.
   (D) The method according to Item (C), wherein a method for the administering is intravitreal injection.
   (E) 5-{5-[3-(Trifluoromethyl)-4-{[(2S)-1,1,1-trifluoropropan-2-yl] oxy}phenyl]-1,2,4-oxadiazol-3-yl}-1H-benzimidazole or a pharmaceutically acceptable salt thereof, for use in prevention or treatment of exudative age-relatedmacular degeneration, diabetic retinopathy, diabetic macular edema, retinopathy of prematurity, myopic choroidal neovascularization, secondary choroidal neovascularization, retinal artery occlusion, retinal vein occlusion, neovascular glaucoma, retinitis pigmentosa, or edema caused by retinal photocoagulation.
   (F) The compound or the pharmaceutically acceptable salt thereof according to Item (E), wherein the compound or the pharmaceutically acceptable salt thereof is for use in intravitreal injection.
   (G) A use of 5-{5-[3-(trifluoromethyl)-4-{[(2S)-1,1,1-trifluoropropan-2-yl] oxy}phenyl]-1,2,4-oxadiazol-3-yl}-1H-benzimidazole or a pharmaceutically acceptable salt thereof, for manufacture of the pharmaceutical composition of Item (A).
   (H) The use according to Item (G), wherein the pharmaceutical composition is for use in intravitreal injection.

### Advantageous Effects of Invention

The selective S1P receptor agonist described in the present application can be used as a preventive or therapeutic agent for an ophthalmic disease associated with intraocular neovascularization and/or increased intraocular vascular permeability, specifically, for example, exudative age-related macular degeneration, diabetic retinopathy, diabetic macular edema, retinopathy of prematurity, myopic choroidal neovascularization, secondary choroidal neovascularization, retinal artery occlusion, retinal vein occlusion, neovascular glaucoma, retinitispigmentosa, or edema caused by retinal photocoagulation.

### Brief Description of Drawings

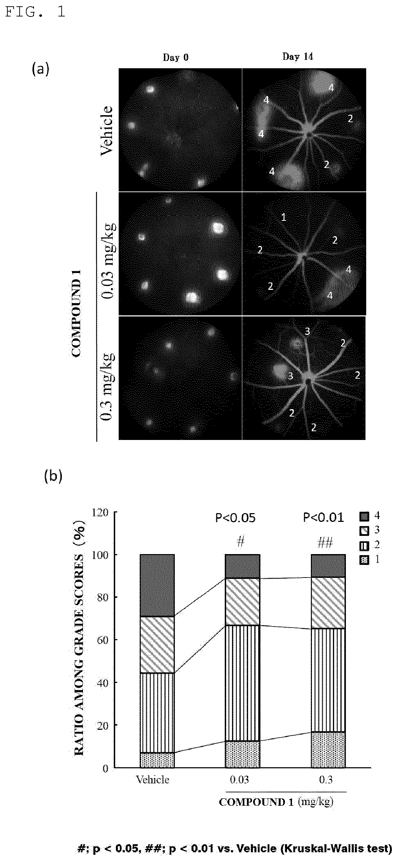

FIG. 1(a) includes fluorescence fundus photography images in Example 1, and numbers in the images represent fluorescein angiography (FA) grades. FIG. 1(b) is a graph showing the FA grades of the fluorescence fundus photography images in Example 1, in which the vertical axis represents a ratio among FA grades of from 1 to 4, and the horizontal axis represents the dose (mg/kg) of Compound 1.

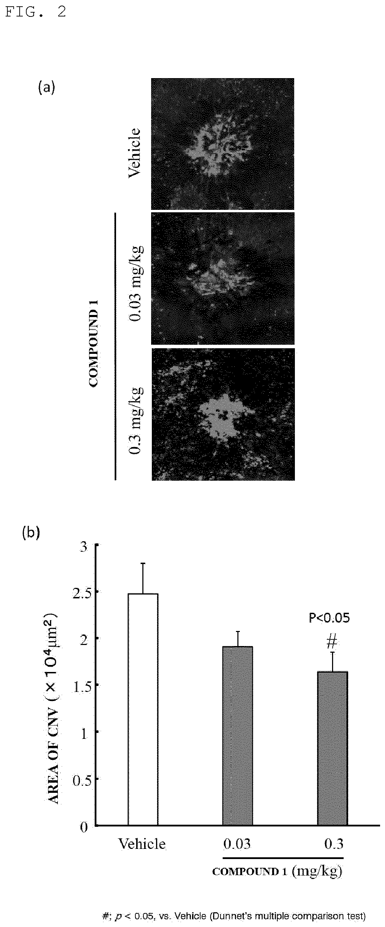

FIG. 2(a) includes images of choroidal flat mount specimens in Example 1. FIG. 2(b) is a graph showing the areas of choroidal neovascularization (CNV) of the choroidal flat mount specimens, in which the vertical axis represents the area of CNV (×10⁴ µm²), and the horizontal axis represents the dose (mg/kg) of Compound 1.

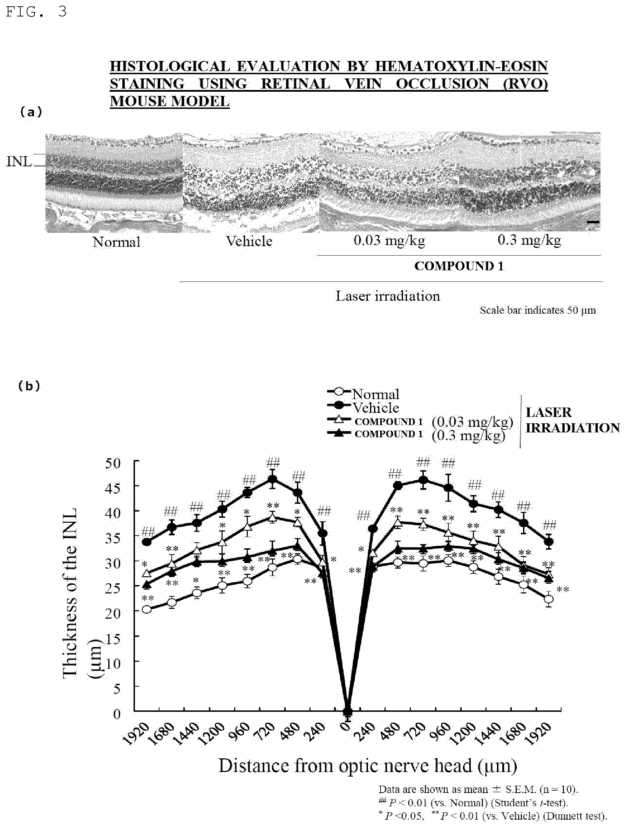

FIG. 3(a) includes histologically stained images in Example 2. FIG. 3(b) is a graph showing the measurement results of the thickness of a retinal inner nuclear layer (INL) in Example 2, in which the vertical axis represents the thickness (µm) of the INL, and the horizontal axis represents a distance (µm) from the center of the optic nerve head.

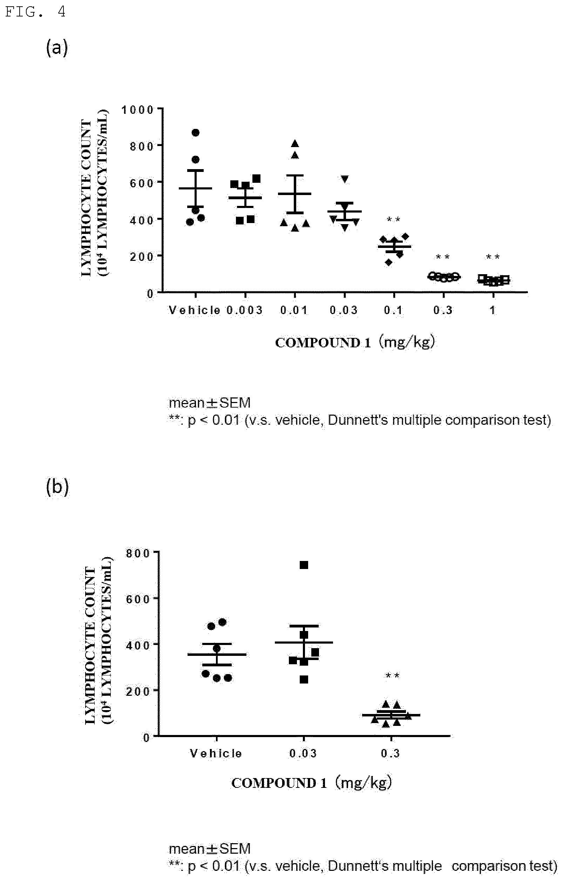

FIG. 4(a) is a graph showing a lymphocyte concentration at a time when Compound 1 was administered to C57BL/6J strain mice in Reference Example 1, in which the vertical axis represents the lymphocyte concentration (×10⁴ lymphocytes/mL), and the horizontal axis represents the dose (mg/kg) of Compound 1. FIG. 4(b) is a graph showing a lymphocyte concentration at a time when Compound 1 was administered to ddy strain mice in Reference Example 1, in which the vertical axis represents the lymphocyte concentration (×10⁴ lymphocytes/mL), and the horizontal axis represents the dose (mg/kg) of Compound 1.

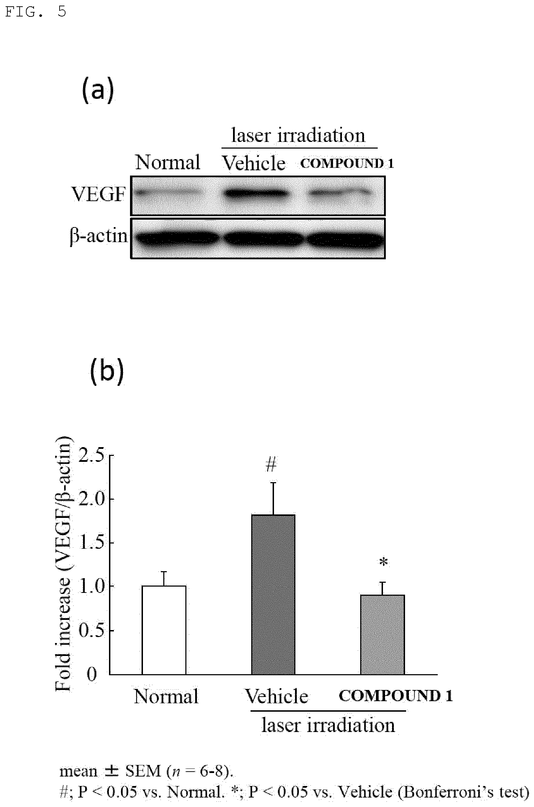

FIG. 5(a) includes western blot images in Example 3. FIG. 5(b) is a graph showing the expression amount of VEGF relative to that of β-actin in Example 3, in which the vertical axis represents the relative value (-fold) of the expression amount of VEGF with reference to a normal group.

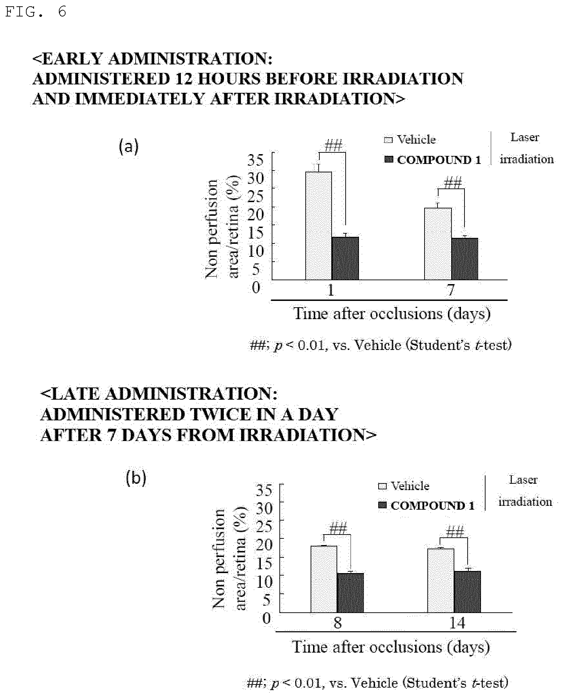

FIGS. 6 show effects on the non perfusion area of a retinal vein occlusion (RVO) model in Example 4. FIG. 6(a) shows results at a time when Compound 1 was administered early after the generation of the model, and FIG. 6(b) shows results at a time when Compound 1 was administered late after the generation of the model. The results show that Compound 1 significantly reduced the enlargement of the non perfusion area in both the early administration and the late administration.

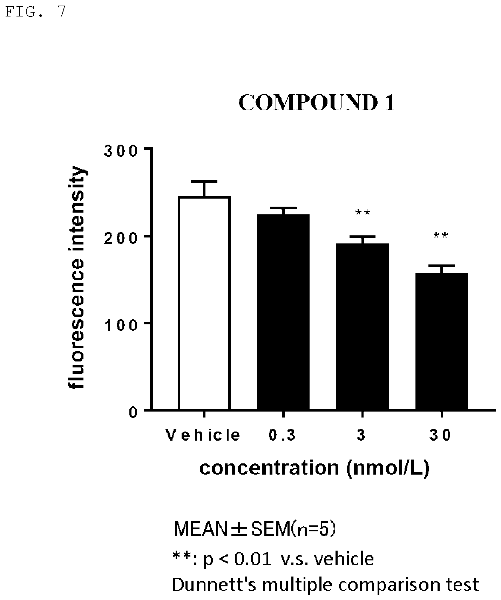

FIG. 7 shows the results of the permeability-reducing action of Compound 1 on human retinal vascular endothelial cells in Example 5. The vertical axis represents the amount of FITC-dextran permeating a lower chamber in terms of fluorescence intensity, and it is shown that Compound 1 significantly decreased the amount of FITC-dextran permeating the lower chamber.

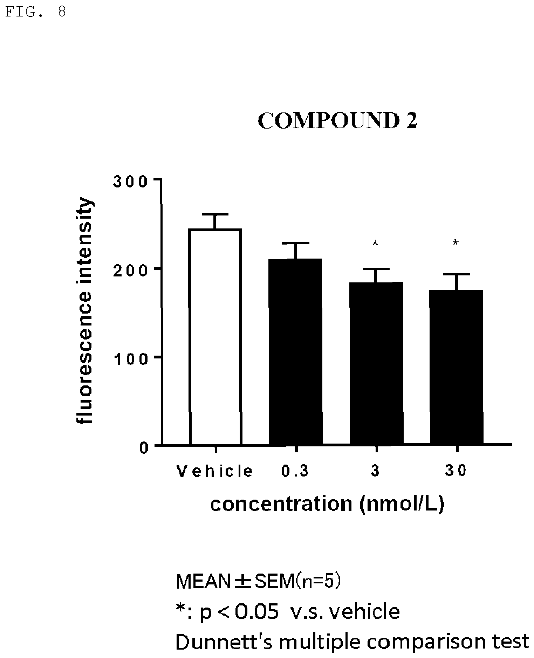

FIG. 8 shows the results of the permeability-reducing action of Compound 2 on human retinal vascular endothelial cells in Example 6. It is shown that Compound 2 significantly decreased the amount of FITC-dextran permeating the lower chamber.

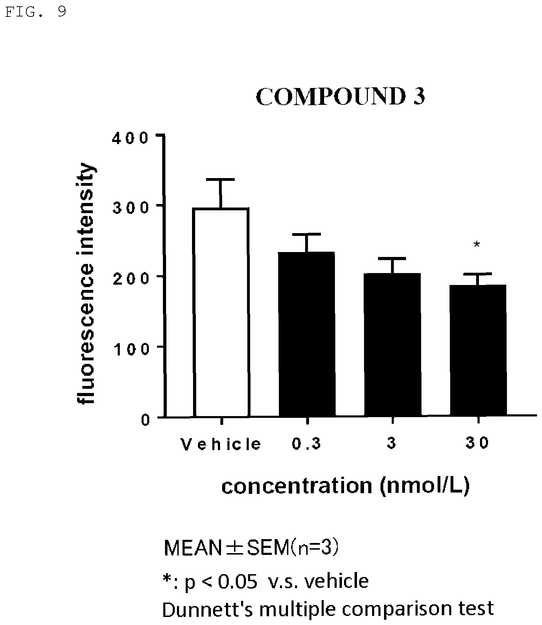

FIG. 9 shows the results of the permeability-reducing action of Compound 3 on human retinal vascular endothelial cells in Example 7. It is shown that Compound 3 significantly decreased the amount of FITC-dextran permeating the lower chamber.

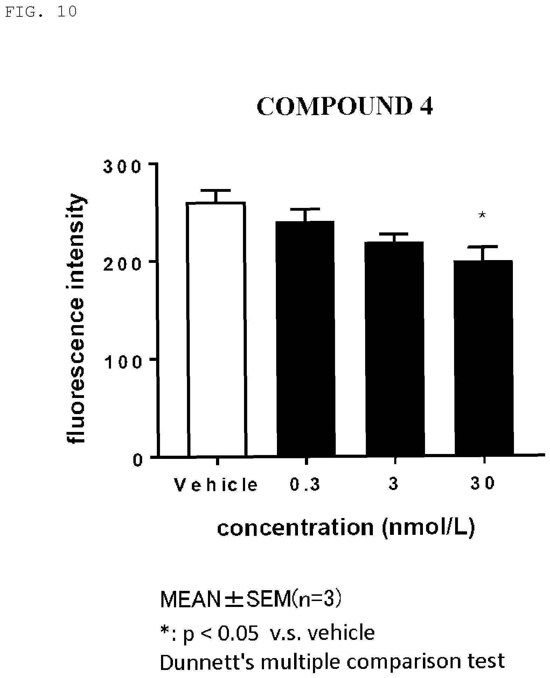

FIG. 10 shows the results of the permeability-reducing action of Compound 4 on human retinal vascular endothelial cells in Example 8. It is shown that Compound 4 significantly decreased the amount of FITC-dextran permeating the lower chamber.

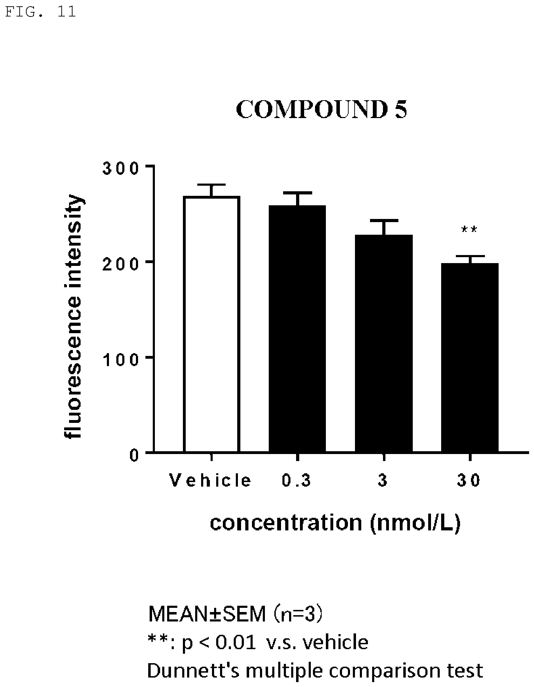

FIG. 11 shows the results of the permeability-reducing action of Compound 5 on human retinal vascular endothelial cells in Example 9. It is shown that Compound 5 significantly decreased the amount of FITC-dextran permeating the lower chamber.

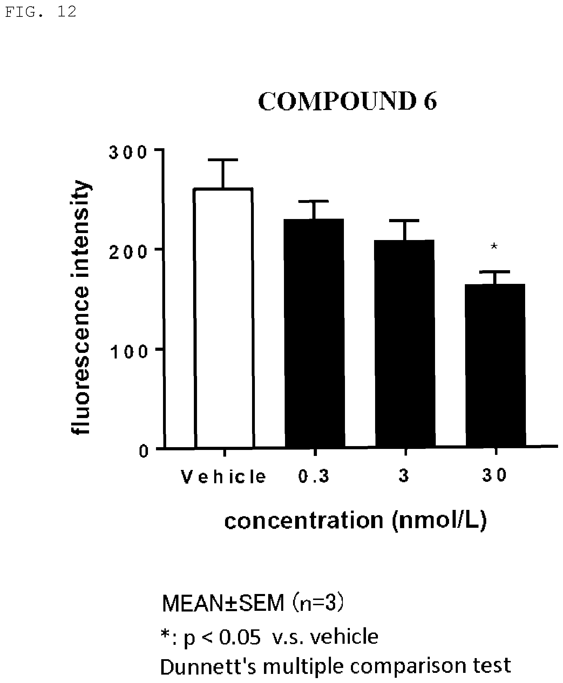

FIG. 12 shows the results of the permeability-reducing action of Compound 6 on human retinal vascular endothelial cells in Example 10. It is shown that Compound 6 significantly decreased the amount of FITC-dextran permeating the lower chamber.

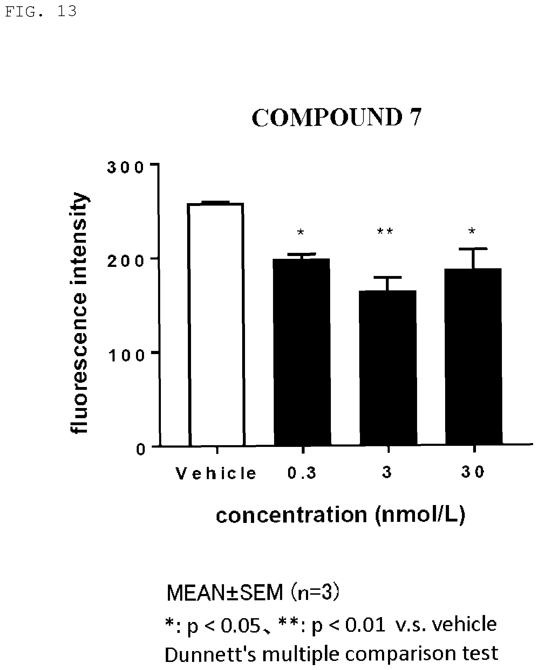

FIG. 13 shows the results of the permeability-reducing action of Compound 7 on human retinal vascular endothelial cells in Example 11. It is shown that Compound 7 significantly decreased the amount of FITC-dextran permeating the lower chamber.

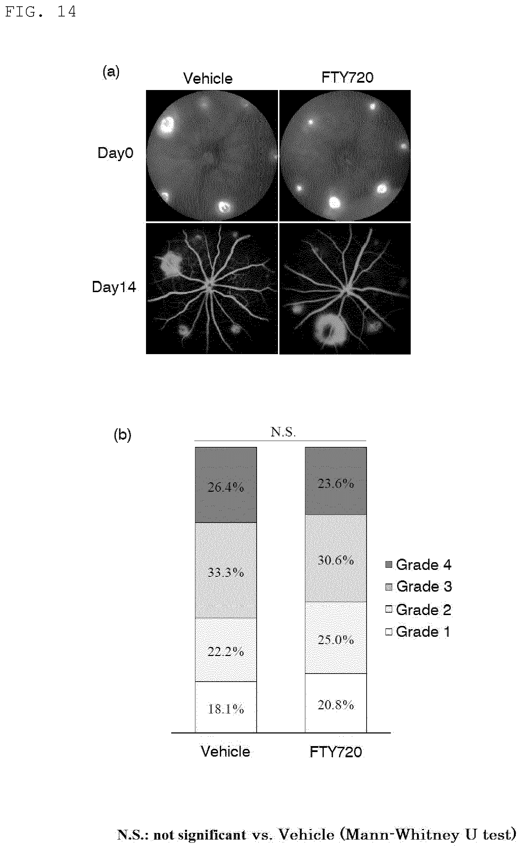

FIG. 14(a) includes fluorescence fundus photography images at a time when FTY720 was administered as Comparative Example 1. FIG. 14(b) is a graph showing the FA grades of the fluorescence fundus photography images of Comparative Example 1, in which the vertical axis represents a ratio among FA grades of from 1 to 4.

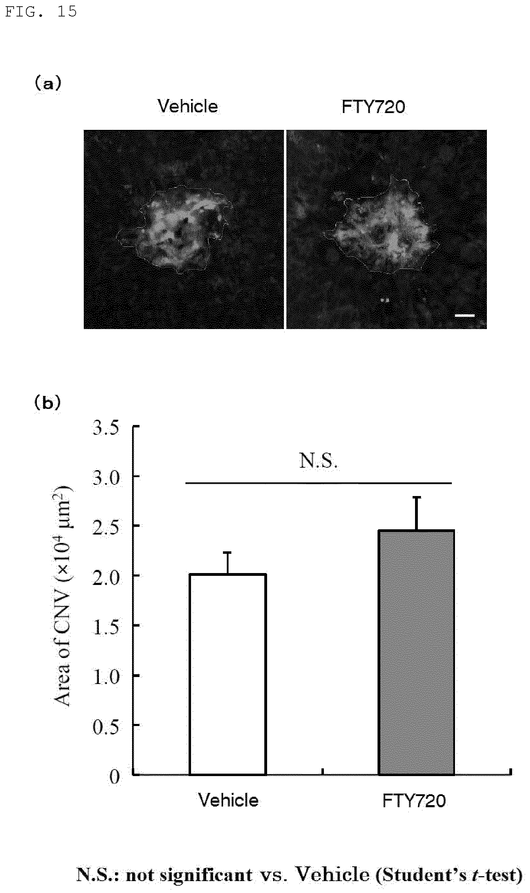

FIG. 15 (a) includes an image of a choroidal flat mount specimen at a time when FTY720 was administered as Comparative Example 1. FIG. 15 (b) is a graph showing the area of choroidal neovascularization (CNV) of the choroidal flat mount specimen.

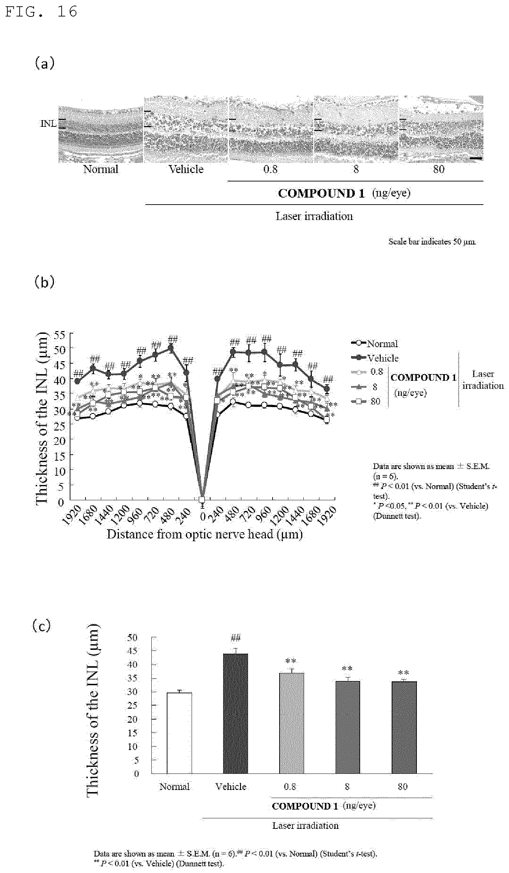

FIG. 16(a) includes histologically stained images in Example 12. FIG. 16(b) is a graph showing the measurement results of the thickness of the INL in Example 12, in which the vertical axis represents the thickness (µm) of the INL, and the horizontal axis represents a distance (µm) from the center of the optic nerve head. FIG. 16(c) is a graph showing the mean of the thicknesses of the INL in Example 12.

### Description of Embodiments

The present invention is described in detail below. Herein, the following abbreviations or symbols are sometimes used.
Compound 1= 5-{5-[3-(trifluoromethyl)-4-{[(2S)-1,1,1-trifluoropropan-2-yl] oxy}phenyl]-1,2,4-oxadiazol-3-yl}-1H-benzimidazole monohydrochloride (Patent Literature 2).
Compound 2= 1-[(7-{[4-(2,2,2-trifluoroethoxy)-3-(trifluoromethyl)phenyl]me thoxy}-2H-1-benzopyran-3-yl)methyl]piperidine-4-carboxylic acid monohydrochloride (Patent Literature 3).
Compound 3= 1-({4-[(1E)-N-{[4-cyclohexyl-3-(trifluoromethyl)phenyl]methoxy }ethanimidoyl]-2-ethylphenyl}methyl)azetidine-3-carboxylic acid (another name: Siponimod or BAF312, CAS No.: 1230487-00-9).
Compound 4= 5-(3-{(1S)-1-[(2-hydroxyethyl)amino]-2,3-dihydro-1H-inden-4-yl }-1,2,4-oxadiazol-5-yl)-2-[(propan-2-yl)oxy]benzonitrile (another name: Ozanimod, CAS No.: 1306760-87-1).
Compound 5= (2Z,5Z)-5-({3-chloro-4-[(2R)-2,3-dihydroxypropoxy]phenyl}methy lidene)-3-(2-methylphenyl)-2-(propylimino)-1,3-thiazolidin-4-o ne (another name: Ponesimod, CAS No.: 854107-55-4).
Compound 6= [(3R)-7-{[4-cyclopentyl-3-(trifluoromethyl)phenyl]methoxy}-1,2 ,3,4-tetrahydrocyclopenta[b]indol-3-yl]acetic acid (another name: Etrasimod, CAS No.: 1206123-37-6).
Compound 7= 2-amino-4-(4-{ [3-(benzyloxy)phenyl]sulfanyl}-2-chlorophenyl)-2 -(hydroxymethyl)butyl dihydrogen phosphate (another name: KRP-203-P, CAS No.: 749262-82-6).
KRP-203=2-amino-2-[2-(4-{[3-(benzyloxy)phenyl]sulfanyl}-2-chlo rophenyl)ethyl]propane-1,3-diol monohydrochloride (CAS No.: 509088-69-1).
FTY720=2-amino-2-[2-(4-octylphenyl)ethyl]propane-1,3-diol monohydrochloride (another name: Fingolimodhydrochloride, CAS No.: 162359-56-0).
FTY720-P=2-amino-2-(hydroxymethyl)-4-(4-octylphenyl)butyl dihydrogen phosphate (another name: FTY720 phosphoric acidmonoester or Fingolimod phosphoric acid monoester, CAS No.: 402615-91-2).

As used herein, the term "agonist" means a compound that binds to a receptor in a manner similar to that of a ligand in a living body, to thereby promote an action, or a pharmaceutically acceptable salt thereof.

As used herein, the term "S1P receptor agonist" means, for example, a compound that binds to an S1P receptor to promote GTP[γ³⁵S] binding to the S1P receptor, or a pharmaceutically acceptable salt thereof.

As used herein, the term "inverse agonist" means a compound that binds to a receptor like an agonist but has an action opposite to that of the agonist, or a pharmaceutically acceptable salt thereof. That is, the term refers to an agent having an opposite action on a receptor, which is different from an agonist.

As usedherein, the term "selective S1P receptor agonist" refers to a compound that selectively has agonist activity at an S1P₁ receptor, or a pharmaceutically acceptable salt thereof. The term "selective" means having agonist activity at the S1P₁ receptor and showing a 10-fold or higher value for agonist activity at an S1P₃ receptor with respect to the agonist activity at the S1P₁ receptor in a comparison in terms of EC₅₀ value in GTP[γ³⁵S] binding assay. The value is, for example, 20-fold or higher in one embodiment, 50-fold or higher in one embodiment, or 100-fold or higher in one embodiment. In addition, the selective S1P receptor agonist may have inverse agonist activity, instead of agonist activity, at the S1P₃ receptor. The selective S1P receptor agonist also encompasses a so-called prodrug that is pharmacologically acceptable. The so-called prodrug that is pharmacologically acceptable is a compound having a group capable of being converted into, for example, a hydroxy group of the compound through solvolysis or under physiological conditions. An example thereof is KRP-203. KRP-203 is phosphorylated into KRP-203-P in a living body.

The EC₅₀ values of Compounds 1 to 6 for S1P₁, S1P₂, S1P₃, S1P₄, and S1P₅ receptor agonist activities in GTP [γ³⁵S] binding assay are as described below.

There is a report that the EC₅₀ values of Compound 1 for S1P₁ receptor agonist activity and S1P₅ receptor agonist activity in GTP [γ³⁵S] binding assay are 7.4 nM and 7.5 nM, respectively, while its EC₅₀ values for agonist activity for each of S1P₂, S1P₃, and S1P₄ receptors are 100-fold or higher values as compared to that for the S1P₁ receptor (PLoS One, 2014, 9(10), e110819).

The EC₅₀ value of Compound 2 for S1P₁ receptor agonist activity in GTP[γ³⁵S] binding assay is 13.4 nM, while its EC₅₀ value for S1P₃ receptor agonist activity is 1.76 µM, which is a 100-fold or higher value as compared to that for the S1P₁ receptor.

The EC₅₀ values of Compounds 3 to 6 for S1P₁, S1P₂, S1P₃, S1P₄, and S1P₅ receptor agonist activities are each described in the following literatures.

Compound 3, Compound 4 (Ozanimod), and Compound 7 (KRP-203-P) : British Journal of Pharmacology, 2016, 173, p. 1778-1792.

Compound 5 (Ponesimod): The Journal of Pharmacology and Experimental Therapeutics, 2011, 337, p. 547-556.

Compound 6 (Etrasimod) : ACS Medicinal Chemistry Letters, 2014, 5, p. 1313-1317.

As used herein, the term "non-selective S1P receptor agonist" means having agonist activity at the S1P₁ receptor and having equal or less than 3-fold agonist activity at the S1P₃ receptor with respect to the agonist activity at the S1P₁ receptor, in a comparison in terms of EC₅₀ value in GTP [γ³⁵S] binding assay. An example thereof is FTY720 or FTY720-P.

There is a report that the EC₅₀ values of FTY720-P for S1P₁, S1P₃, S1P₄, and S1P₅ receptor agonist activities in GTP[γ³⁵S] binding assay are 1.4 nM, 2.9 nM, 2.2 nM, and 0.86 nM, respectively, showing the presence of agonist activity at the S1P₃ receptor (PLoS One, 2014, 9(10), e110819).

As used herein, the terms "compound having agonist activity at the S1P₁ receptor, or a pharmaceutically acceptable salt thereof", "compound having agonist activity at the S1P₄ receptor, or a pharmaceutically acceptable salt thereof" , and "compound having agonist activity at the S1P₅ receptor, or a pharmaceutically acceptable salt thereof" refer to a compound that binds to the S1P₁ receptor, the S1P₄ receptor, or the S1P₅ receptor, respectively, to promote GTP [γ³⁵S] binding to the receptor, or a pharmaceutically acceptable salt thereof. For example, the compound having agonist activity at the S1P₁ receptor, or the pharmaceutically acceptable salt thereof is, in one embodiment, a compound having an EC₅₀ value of from 100 µM to 1 pM for S1P₁ receptor agonist action in GTP[γ³⁵S] binding assay, or a pharmaceutically acceptable salt thereof, is, in one embodiment, a compound having an EC₅₀ value of from 100 nM to 10 pM, or a pharmaceutically acceptable salt thereof, and is, in one embodiment, a compound having an EC₅₀ value of from 10 nM to 0.1 nM, or a pharmaceutically acceptable salt thereof. The same applies to the "compound having agonist activity at the S1P₄ receptor, or a pharmaceutically acceptable salt thereof" and the "compound having agonist activity at the S1P₅ receptor, or a pharmaceutically acceptable salt thereof". A method known to a person skilled in the art as described in, for example, PLoS One, 2014, 9(10), e110819 may be used as a method of measuring an EC₅₀ value for S1P receptor agonist action in GTP[γ³⁵S] binding assay.

The selective S1P receptor agonist described in the present application encompasses:
a) a compound having agonist activity at the S1P₁ receptor, or a pharmaceutically acceptable salt thereof;
b) a compound having agonist activity at each of the S1P₁ receptor and the S1P₄ receptor, or a pharmaceutically acceptable salt thereof;
c) a compound having agonist activity at each of the S1P₁ receptor and the S1P₅ receptor, or a pharmaceutically acceptable salt thereof; and
d) a compound having agonist activity at each of the S1P₁ receptor, the S1P₄ receptor, and the S1P₅ receptor, or a pharmaceutically acceptable salt thereof. In one embodiment, the compound having each agonist activity is a compound showing an EC₅₀ value of from 100 µM to 1 pM for each S1P receptor agonist action in GTP[γ³⁵S] binding assay, or a pharmaceutically acceptable salt thereof.

With regard to agonist activity at each of the S1P₂ receptor and the S1P₃ receptor (hereinafter referred to as "other S1P receptors"), in one embodiment, the agonist activity at each of the other S1P receptors is 10-fold or higher with respect to the agonist activity at the S1P₁ receptor in a comparison in terms of EC₅₀ value in GTP[γ³⁵S] binding assay. In one embodiment, the value is 20-fold or higher. In another embodiment, the value is 50-fold or higher. In still another embodiment, the value is 100-fold or higher.

The term "other S1P receptors", which refers to the S1P₂ receptor and the S1P₃ receptor, refers to the S1P₃ receptor in one embodiment.

Embodiments of the selective S1P receptor agonist described in the present application are described below.
1) A compound having agonist activity at the S1P₁ receptor, or a pharmaceutically acceptable salt thereof.
2) A compound having agonist activity at each of the S1P₁ receptor and the S1P₄ receptor, or a pharmaceutically acceptable salt thereof .
3) A compound having agonist activity at each of the S1P₁ receptor and the S1P₅ receptor, or a pharmaceutically acceptable salt thereof .
4) A compound having agonist activity at each of the S1P₁ receptor, the S1P₄ receptor, and the S1P₅ receptor, or a pharmaceutically acceptable salt thereof.
5) A compound having agonist activity at the S1P₁ receptor and showing a 100-fold or higher value for agonist activity at each of the other S1P receptors with respect to the agonist activity at the S1P₁ receptor in a comparison in terms of EC₅₀ value in GTP[γ³⁵S] binding assay, or a pharmaceutically acceptable salt thereof.
6) A compound having agonist activity at each of the S1P₁ receptor and the S1P₄ receptor and showing a 100-fold or higher value for agonist activity at each of the other S1P receptors with respect to the agonist activity at the S1P₁ receptor in a comparison in terms of EC₅₀ value in GTP[γ³⁵S] binding assay, or a pharmaceutically acceptable salt thereof.
7) A compound having agonist activity at each of the S1P₁ receptor and the S1P₅ receptor and showing a 100-fold or higher value for agonist activity at each of the other S1P receptors with respect to the agonist activity at the S1P₁ receptor in a comparison in terms of EC₅₀ value in GTP[γ³⁵S] binding assay, or a pharmaceutically acceptable salt thereof.
8) A compound having agonist activity at each of the S1P₁ receptor, the S1P₄ receptor, and the S1P₅ receptor and showing a 100-fold or higher value for agonist activity at each of the other S1P receptors with respect to the agonist activity at the S1P₁ receptor in a comparison in terms of EC₅₀ value in GTP[γ³⁵S] binding assay, or a pharmaceutically acceptable salt thereof.
9) A compound showing a 10-fold or higher value in the comparison in terms of EC₅₀ value in any one of Items 5) to 8), or a pharmaceutically acceptable salt thereof.
10) A compound having agonist activity at the S1P₁ receptor as compared to the S1P₃ receptor, or a pharmaceutically acceptable salt thereof.
11) A compound having agonist activity at each of the S1P₁ receptor and the S1P₄ receptor as compared to the S1P₃ receptor, or a pharmaceutically acceptable salt thereof.
12) A compound having agonist activity at each of the S1P₁ receptor and the S1P₅ receptor as compared to the S1P₃ receptor, or a pharmaceutically acceptable salt thereof.
13) A compound having agonist activity at each of the S1P₁ receptor, the S1P₄ receptor, and the S1P₅ receptor as compared to the S1P₃ receptor, or a pharmaceutically acceptable salt thereof.
14) A compound having agonist activity at the S1P₁ receptor and showing a 10-fold or higher value for agonist activity at the S1P₃ receptor with respect to the agonist activity at the S1P₁ receptor in a comparison in terms of EC₅₀ value in GTP[γ³⁵S] binding assay, or a pharmaceutically acceptable salt thereof.
15) A compound having agonist activity at each of the S1P₁ receptor and the S1P₄ receptor and showing a 10-fold or higher value for agonist activity at the S1P₃ receptor with respect to the agonist activity at the S1P₁ receptor in a comparison in terms of EC₅₀ value in GTP [γ³⁵S] binding assay, or a pharmaceutically acceptable salt thereof.
16) A compound having agonist activity at each of the S1P₁ receptor and the S1P₅ receptor and showing a 10-fold or higher value for agonist activity at the S1P₃ receptor with respect to the agonist activity at the S1P₁ receptor in a comparison in terms of EC₅₀ value in GTP [γ³⁵S] binding assay, or a pharmaceutically acceptable salt thereof.
17) A compound having agonist activity at each of the S1P₁ receptor, the S1P₄ receptor, and the S1P₅ receptor and showing a 10-fold or higher value for agonist activity at the S1P₃ receptor with respect to the agonist activity at the S1P₁ receptor in a comparison in terms of EC₅₀ value in GTP [γ³⁵S] binding assay, or a pharmaceutically acceptable salt thereof.
18) A compound showing a 50-fold or higher value in the comparison in terms of EC₅₀ value in any one of Items 14) to 17), or a pharmaceutically acceptable salt thereof.
19) A compound showing a 100-fold or higher value in the comparison in terms of EC₅₀ value in any one of Items 14) to 17), or a pharmaceutically acceptable salt thereof.
   Examples of the selective S1P receptor agonist according to one embodiment of the invention of the present application include the following compounds or pharmaceutically acceptable salts thereof.
20) 5-{5-[3-(Trifluoromethyl)-4-{[(2S)-1,1,1-trifluoropropan-2-yl] oxy}phenyl]-1,2,4-oxadiazol-3-yl}-1H-benzimidazole or a pharmaceutically acceptable salt thereof, 1-[(7-{[4-(2,2,2-trifluoroethoxy)-3-(trifluoromethyl)phenyl]me thoxy}-2H-1-benzopyran-3-yl)methyl]piperidine-4-carboxylic acid or a pharmaceutically acceptable salt thereof, 1-({4-[(1E)-N-{[4-cyclohexyl-3-(trifluoromethyl)phenyl]methoxy }ethanimidoyl]-2-ethylphenyl}methyl)azetidine-3-carboxylic acid or a pharmaceutically acceptable salt thereof, 5-(3-{(1S)-1-[(2-hydroxyethyl)amino]-2,3-dihydro-1H-inden-4-yl }-1,2,4-oxadiazol-5-yl)-2-[(propan-2-yl)oxy]benzonitrile or a pharmaceutically acceptable salt thereof, (2Z,5Z)-5-({3-chloro-4-[(2R)-2,3-dihydroxypropoxy]phenyl}methy lidene)-3-(2-methylphenyl)-2-(propylimino)-1,3-thiazolidin-4-o ne or a pharmaceutically acceptable salt thereof, [(3R)-7-{[4-cyclopentyl-3-(trifluoromethyl)phenyl]methoxy}-1,2 ,3,4-tetrahydrocyclopenta[b]indol-3-yl]acetic acid or a pharmaceutically acceptable salt thereof, and 2-amino-2-[2-(4-{[3-(benzyloxy)phenyl]sulfanyl}-2-chlorophenyl )ethyl]propane-1,3-diol or a pharmaceutically acceptable salt thereof.
21) 5-{5-[3-(Trifluoromethyl)-4-{[(2S)-1,1,1-trifluoropropan-2-yl] oxy}phenyl]-1,2,4-oxadiazol-3-yl}-1H-benzimidazole monohydrochloride, 1-[(7-{[4-(2,2,2-trifluoroethoxy)-3-(trifluoromethyl)phenyl]me thoxy}-2H-1-benzopyran-3-yl)methyl]piperidine-4-carboxylic acid monohydrochloride, 1-({4-[(1E)-N-{[4-cyclohexyl-3-(trifluoromethyl)phenyl]methoxy }ethanimidoyl]-2-ethylphenyl}methyl)azetidine-3-carboxylic acid (Siponimod), 5-(3-{(1S)-1-[(2-hydroxyethyl)amino]-2,3-dihydro-1H-inden-4-yl }-1,2,4-oxadiazol-5-yl)-2-[(propan-2-yl)oxy]benzonitrile (Ozanimod), (2Z,5Z)-5-({3-chloro-4-[(2R)-2,3-dihydroxypropoxy]phenyl}methy lidene)-3-(2-methylphenyl)-2-(propylimino)-1,3-thiazolidin-4-o ne (Ponesimod), [(3R)-7-{[4-cyclopentyl-3-(trifluoromethyl)phenyl]methoxy}-1,2 ,3,4-tetrahydrocyclopenta[b]indol-3-yl]acetic acid (Etrasimod), and 2-amino-2-[2-(4-{ [3-(benzyloxy)phenyl]sulfanyl}-2-chlorophenyl )ethyl]propane-1,3-diol monohydrochloride (KRP-203).
(22) 5-{5-[3-(Trifluoromethyl)-4-{[(2S)-1,1,1-trifluoropropan-2-yl] oxy}phenyl]-1,2,4-oxadiazol-3-yl}-1H-benzimidazole or a pharmaceutically acceptable salt thereof.

As used herein, the "pharmaceutically acceptable salt" refers to a pharmaceutically acceptable salt of a compound, and depending on the kind of a substituent thereof, an acid addition salt or a salt with a base may be formed. Specific examples thereof include: acid addition salts with inorganic acids, such as hydrochloric acid, hydrobromic acid, hydroiodic acid, sulfuric acid, nitric acid, and phosphoric acid, and organic acids, such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditoluoyltartaric acid, citric acid, methanesulfonic acid, ethanesulfonic acid, benzenesulfonic acid, p-toluenesulfonic acid, aspartic acid, and glutamic acid; salts with inorganic bases, such as sodium, potassium, magnesium, calcium, and aluminum, and organic bases, such as methylamine, ethylamine, ethanolamine, lysine, and ornithine; salts with various amino acids and amino acid derivatives, such as acetylleucine; and an ammonium salt.

A pharmaceutical composition of the present invention is prepared using a carrier, an excipient, and other additives that are generally used for drug formulation. The pharmaceutical composition may be administered by oral administration in any form, such as a tablet, a pill, a capsule, a granule, a powder, or a solution, or by parenteral administration in any form, for example, an injection, such as an intravenous injection, an intramuscular, an intravitreal injection, or a sub-Tenon's capsule injection. In one embodiment, oral administration is adopted. In one embodiment, intraocular administration is adopted. In one embodiment, intravitreal administration is adopted. In one embodiment, intraocular injection or intravitreal injection is adopted. In one embodiment, intraocular injection is adopted. In one embodiment, intravitreal injection is adopted.

A dose is determined as appropriate for individual cases in consideration of, for example, symptoms, and the age or sex of an administration subject. However, in the case of oral administration, a daily dose for adults is generally selected from the range of from 0.0001 mg/kg to 100 mg/kg, preferably from 0.001 mg/kg to 0.3 mg/kg, and this amount may be administered in a single dose or two to four divided doses. In addition, in the case where intravenous administration is performed due to symptoms, a single dose for adults in the range of from 0.0001 mg/kg to 10 mg/kg, preferably from 0.001 mg/kg to 0.3 mg/kg is generally administered once or a plurality of times a day.

In the case of intravitreal administration, a single dose for adults in the range of from 0.1 ng to 5 mg, preferably from 20 ng to 1 mg is generally administered once or a plurality of times (i.e., 2, 3, 4, 5, 6 or more times) . The plurality of times of administration may be performed at constant intervals or irregular intervals. The timing of administration may be before the onset of a disease or after the onset. In one embodiment, the administration before the onset is performed, for example, 12 hours before the onset. In one embodiment, the administration is performed, for example, 1 day before the onset. In one embodiment, the timing of the administration after the onset is selected from the group consisting of: immediately after the onset; about 1 week after the onset; 2 weeks after the onset; 1 month after the onset; 6 weeks after the onset; 3 months after the onset; and after a period longer than 3 months from the onset. In one embodiment, the constant intervals are each about 3 months. In one embodiment, the timing is monthly for 6 consecutive months after the onset.

As a solid composition for oral administration according to the present invention, a tablet, a powder, a granule, or the like is used. In such solid composition, one or more active substances may be mixed with at least one inert excipient or the like. The composition may contain an inert additive, such as a lubricant, a disintegrant, or a dissolution aid, in accordance with a conventional method. A tablet or a pill may be subjected to sugarcoating or gastric or enteric coating as required.

As a liquid composition for oral administration, an emulsion, a solution, a suspension, a syrup, an elixir, or the like is used. The liquid composition contains a generally used inert solvent, for example, purified water or ethanol. This composition may contain, in addition to the inert solvent, a pharmaceutical aid, such as a solubilizer, a humectant, or a suspending agent, a sweetening agent, a taste-masking agent, a flavoring agent, and a preservative.

A pharmaceutical for preventing or treating an ophthalmic disease of the present invention may be parenterally administered. Examples of its dosage form include an ophthalmic ointment and an injection.

An injection for parenteral administration contains a sterile aqueous or non-aqueous solution, suspension, or emulsion. The injection contains, for example, distilled water for injection or physiological saline as an aqueous solvent. As a non-aqueous solvent, there is given, for example, an alcohol, such as ethanol. Such composition may further contain a tonicity agent, a preservative, a humectant, an emulsifier, a dispersant, a stabilizer, or a dissolution aid. Such composition is sterilized by, for example, filtration through a bacteria-retaining filter, blending of a germicide, or irradiation. In addition, such composition may also be used as follows: a sterile solid composition is manufactured, and dissolved or suspended in sterile water or a sterile vehicle for injection before use.

The disease to be targeted in the present invention is an ophthalmic disease associated with intraocular neovascularization and/or increased intraocular vascular permeability. Specifically, the present invention is applied to, for example, exudative age-related macular degeneration, diabetic retinopathy, diabetic macular edema, retinopathy of prematurity, myopic choroidal neovascularization, secondary choroidal neovascularization, retinal artery occlusion, retinal vein occlusion, neovascular glaucoma, retinitis pigmentosa, or edema caused by retinal photocoagulation. In addition, specifically, the present invention is desirably applied to, for example, exudative age-relatedmacular degeneration, diabetic retinopathy, or diabetic macular edema.

The agent of the present invention may be administered together with another therapeutic agent. For example, the other agent may be an analgesic, an anesthetic, an immunosuppressant, or an anti-inflammatory agent. The agent of the present invention may be administered together with specifically an anti-VEGF agent, more specifically an anti-VEGF antibody. For combined administration with the other agent, both agents may be administered together in a single composition, or may be administered in separate compositions as part of a combined therapy.

### Examples

In order to understand the present invention deeper, the experimental results leading to the present invention are shown in each of Reference Examples, specific contents of the invention are shown in Examples, and they are described in detail. The present invention is not limited to the matters described in these Reference Examples or Examples.

### (Example 1) Effects on Laser-induced Choroidal Neovascularization (CNV) Model

### (1) Evaluation of Fluorescein Angiography (FA) Grade

A Mydrin-P ophthalmic solution (Santen Pharmaceutical Co., Ltd., Mydrin is a trademark) was dropped into the right eye of 8-week-oldmice (Japan SLC, Inc., C56BL/6J strain) to cause mydriasis. A solution obtained by diluting a 7:1 mixed anesthetic solution of ketamine and xylazine 10-fold with physiological saline was administered at 10 mL/kg into the femoral muscle. After that, a 0.1%Hyalein (trademark) ophthalmic solution (Santen Pharmaceutical Co., Ltd.) was dropped into the eye so as to prevent the eyeball from drying.

Then, while a cover glass was put to the right eye, the fundus was looked into, and six points on the periphery of the optic nerve head at equal intervals were subjected to laser irradiation (wavelength: 647 nm, spot size: 50 µm, irradiation time: 100 msec, laser output: 120mW) using a laser beam coagulation apparatus (MC500; NIDEK CO. LTD, Aichi, Japan).

Compound 1 was suspended in a vehicle (0.5% methylcellulose (MC) solution), and was orally administered at a dose of 0.03 mg/kg or 0.3 mg/kg (in terms of free form) once daily from the day before the laser irradiation to the day before sampling. A control was administered only the vehicle (vehicle group). Experiments were performed using 16 mice in each Compound 1 administration group and 15 mice in the vehicle group.

Sampling was performed on the 14th day after the laser irradiation. The mice were anesthetized with 10 mL/kg of a solution obtained by diluting a 7:1 mixed anesthetic solution of ketamine and xylazine 10-fold with physiological saline, and then 0.1 mL of a 10-fold dilution of fluorescein (Alcon Japan Ltd.) was administered into the tail vein of the mice, followed by fluorescence fundus photography using Micron 4 (Phoenix Research Laboratories, Inc. Pleasanton, CA, USA).

The results are shown in FIG. 1(a). In FIG. 1(a), Day 0 indicates images taken on the day of the laser irradiation, and Day 14 indicates images taken on the 14th day. In addition, for the image of each group on Day 14, an FA grade was determined for each laser irradiation site in accordance with grade criteria shown in Table 1 to evaluate the leakage of a fluorescent dye from a neovascularization site. The results are shown in FIG. 1(b).

As apparent from FIG. 1(b), in each of the groups administered Compound 1, the FA grade was found to be significantly lowered as compared to the vehicle group, confirming a reducing effect on blood leakage from new blood vessels. This shows that Compound 1 has a permeability-reducing action on new blood vessels.

**Table 1**

| Grade criteria | |
|---|---|
| Grade 1 | Hyperfluorescence is not found. |
| Grade 2 | No leakage is found, but hyperfluorescence is found. |
| Grade 3 | Leakage and hyperfluorescence are found. |
| Grade 4 | Leakage and hyperfluorescence are found, and the leakage goes beyond the irradiated area. |

### (2) Evaluation of Area of CNV

After fluorescence fundus photography had been performed as described above in the section (1), 0.5 mL of fluorescein isothiocyanate dextran (FITC-dextran; 20 mg/mL, Sigma-Aldrich) was administered into the tail vein of the mice. The mice were euthanized by cervical dislocation, and the eyeball was extirpated. The extirpated eyeball was fixed in a 4% paraformaldehyde phosphate buffer for 12 hours. After that, the cornea and lens were excised under a microscope, and the remaining vitreous artery was removed with forceps. Further, the retina was removed, and the choroid was notched 8 points and embedded with Fluoromount (Diagnostic BioSystems) in a flat state to generate a choroidal flat mount specimen. The choroidal flat mount was photographed using a confocal laser scanning microscope (FLUOVIEW FV10i; Olympus, Tokyo, Japan). The resultant images are shown in FIG. 2(a).

Next, on the basis of the photographed image, the CNV was encircled using analysis software OLYMPUS FLUOVIEW FV10i (Olympus), and the area thereof was determined as the area of CNV (µm²). All analyses were performed in a blinded manner. The results are shown in FIG. 2(b).

In the group administered 0.03 mg/kg of Compound 1, a decrease in area of CNV indicating new blood vessels was found, and particularly in the group administered 0.3 mg/kg of Compound 1, a significant decrease was found. The reduction ratio of the area of CNV of the 0.3 mg/kg administration group was 34%.

The above-mentioned results confirmed that Compound 1 reduced neovascularization in this model.

InExample 1, a significant change in body weight between the groups before sampling was not found. A case in which FA was unable to be performed owing to cloudiness caused by a flaw in the eye produced before the FA evaluation and a case in which CNV was connected at the time of the evaluation of the area of CNV were excluded from analysis, and the above-mentioned results were each calculated as a mean of 11 or 12 mice in each group.

### (Example 2) Effect on Retinal Vein Occlusion (RVO) Model

Eight-week-old mice (Japan SLC, Inc., ddy strain) were anesthetized by intramuscularly administering 10 mL/kg of a mixed anesthetic solution diluted so as to contain ketamine (120 mg/kg) and xylazine (6 mg/kg). After that, 0.15 mL of rose bengal (20 mg/kg; WAKO) was administered into the tail vein, and a vein three head systems away from the optic nerve head of the right eye of the mice was irradiated with a laser to occlude a retinal vein. For the occlusion, the laser irradiation was performed 10 times to 15 times per vein. For the laser irradiation, an attachment of a fundus photography apparatus Micron 4 (Phoenix Research Laboratories, Inc., Pleasanton, CA, USA) was used (wavelength: 532 nm, spot size: 50 µm, irradiation time: 5,000 ms, laser output: 50 mW). Three veins were occluded per eye, and it was confirmed with photographs of the fundus that the laser-irradiated veins had been completely occluded.

Compound 1 was suspended in a vehicle (0.5% MC solution), and was orally administered at a dose of 0.03 mg/kg or 0.3 mg/kg (in terms of free form) 12 hours before the laser irradiation and immediately after the irradiation. Controls were untreated (normal group) and administered only the vehicle (vehicle group). Experiments were performed using 10 mice in each of the Compound 1 administration groups, the normal group, and the vehicle group.

After 24 hours from the laser irradiation, the mice were euthanized by cervical dislocation, the eyeball was extirpated, and histological evaluation was performed by a hematoxylin-eosin staining method. In addition, the thickness of the retinal inner nuclear layer (INL) was measured at intervals of 240 µm on both sides, i.e., ear side and nose side from the center of the optic nerve head. All analyses were performed in a blinded manner. Stained images in the histological evaluation are shown in FIG. 3(a). In addition, the measurement results of the INL thickness are shown in FIG. 3(b).

As apparent from the results shown in FIG. 3(b), it was shown that Compound 1 ameliorated the thickening of the INL at an dose of at least 0.03 mg/kg or more. Thus, it was revealed that Compound 1 ameliorated retinal edema formed in the retinal vein occlusion model. That is, it was confirmed that Compound 1 had a permeability-reducing action on the retinal vascular endothelium.

### (Reference Example 1) Correlation with Lymphocyte-decreasing Action

Seven-week-old mice (Japan SLC, Inc., C57BL/6J strain) were repeatedly orally administered Compound 1 suspended in a vehicle (0.5% MC solution) at a dose of 0.003 mg/kg, 0.01 mg/kg, 0.03 mg/kg, 0.1 mg/kg, 0.3 mg/kg, or 1 mg/kg (in terms of free form) once daily for 14 days in the same manner as in Example 1. A control was administered only the vehicle (vehicle group). Experiments were performed using 5 mice in each group.

At 24 hours from the final administration, blood was collected from the abdominal vena cava under isoflurane anesthesia using a heparin-treated syringe, and was mixed with ethylenediaminetetraacetic acid dipotassium salt (EDTA·2K) (1 mg). The lymphocyte count in the collected blood was measured using XT-2000iv (Sysmex Corporation) . The results are shown in FIG. 4 (a) . Compound 1 significantly decreased lymphocytes in the peripheral blood at 0.1 mg/kg or more, but was not found to have a significant action at 0.03 mg/kg.

Next, 7-week-old mice (Japan SLC, Inc., ddy strain) were orally administered Compound 1 suspended in a vehicle (0.5% MC solution) at a dose of 0.03 mg/kg or 0.3 mg/kg (in terms of free form) twice at an interval of 12 hours in the same manner as in Example 2. A control was administered only the vehicle (vehicle group). Experiments were performed using 6 mice in each group.

After 24 hours from the second administration, blood was collected from the abdominal vena cava under isoflurane anesthesia using a heparin-treated syringe, and was mixed with EDTA·2K (1 mg) . The lymphocyte count in the collected blood was measured using XT-2000iv (Sysmex Corporation) . The results are shown in FIG. 4 (b) .

As shown in FIG. 4(a) and FIG. 4(b), the group administered 0.03 mg/kg of Compound 1 did not cause a significant decrease in lymphocytes in the peripheral blood.

Meanwhile, according to FIG. 1(b) showing the reducing action on blood leakage from new blood vessels in Example 1, and FIG. 3 (b) showing the retinal edema-ameliorating action in Example 2, in each of which Compound 1 was administered by the same method as described above, respective effects were found even in the group administered 0.03 mg/kg of Compound 1.

The above-mentioned results suggest that the neovascularization permeability-reducing action and retinal vascular endothelium permeability-reducing action of Compound 1 do not depend on a lymphocyte-decreasing effect.

### (Example 3) VEGF Production-reducing Effect in CNVModel

A CNV model generated by the same method as in Example 1 was orally administered Compound 1 suspended in a vehicle (0.5% MC solution) at a dose of 0.3 mg/kg (in terms of free form) once daily from the day before laser irradiation to the day of sampling. Controls were an untreated group (normal group) and a group administered only the vehicle (vehicle group). Experiments were performed using 6 mice in the Compound 1 administration group, 8 mice in the normal group, and 7 mice in the vehicle group.

After 5 days from the laser irradiation, the mice were euthanized by cervical dislocation, and the eyeball was extirpated. Choroid-retinal pigment epithelium was isolated from the eyeball, and the expression amount of VEGF protein was measured by a western blot method using an anti-VEGF antibody (manufactured by Merck Millipore). The results of the western blotting are shown in FIG. 5(a). In addition, the results of calculation of the expression amount of VEGF relative to that of β-actin with reference to the normal group are shown in FIG. 5(b).

As shown in FIG. 5(a) and FIG. 5(b), it was confirmed that Compound 1 more significantly reduced the expression of VEGF as compared to the vehicle group. This suggested that Compound 1 achieved the reduction of neovascularization and the reduction of blood leakage from new blood vessels shown in the results of Example 1 because of also having an action of reducing the expression of VEGF playing important roles in development of choroidal neovascularization and increase of vascular permeability, in addition to an action on the S1P₁ receptor on endothelial cells.

### (Example 4) Effect on Non Perfusion Area of Retinal Vein Occlusion (RVO) Model

Eight-week-old mice (Japan SLC, Inc., ddy strain) were anesthetized by intramuscularly administering 10 mL/kg of a mixed anesthetic solution diluted so as to contain ketamine (120 mg/kg) and xylazine (6 mg/kg) . After that, 0.15 mL of rose bengal (20 mg/kg; WAKO) was administered into the tail vein, and a vein three head systems away from the optic nerve head of the right eye of the mice was irradiated with a laser to occlude a retinal vein. Thus, a retinal non perfusion area was generated. For the occlusion, the laser irradiation was performed 10 times to 15 times per vein. For the laser irradiation, an attachment of a fundus photography apparatus Micron 4 (Phoenix Research Laboratories, Inc., Pleasanton, CA, USA) was used (wavelength: 532 nm, spot size: 50 µm, irradiation time: 5, 000 ms, laser output: 50 mW). Three veins were occluded per eye, and it was confirmed with photographs of the fundus that the laser-irradiated veins had been completely occluded.

Compound 1 was suspended in a vehicle (0.5% MC solution), and was orally administered in all cases at a dose of 0.3 mg/kg (in terms of free form) twice, i.e., 12 hours before the laser irradiation and immediately after the irradiation (early administration), or twice at an interval of 12 hours on the 7th day after the laser irradiation (late administration). A control was administered only the vehicle (vehicle group) . Experiments were performed using 10 mice in each of the Compound 1 early administration group, the Compound 1 late administration group, and the vehicle group.

After 1 day and after 7 days from the second administration, the mice were anesthetized by intramuscularly administering 10 mg/kg of a mixed anesthetic solution of ketamine (120 mg/kg) and xylazine (6 mg/kg). A solution of fluorescein isothiocyanate (FITC)-dextran having a molecular weight of 2×10⁶ dissolved in 0.01 M phosphate-buffered saline was administered at 1 mL into the tail vein (corresponding to 20 mg of FITC-dextran) . After 5 minutes, the eyeball was extirpated, and was fixed in a 4% paraformaldehyde-containing 0.1 M phosphate buffer (pH 7.4) for 7 hours. While the fixed eyeball was immersed in phosphate-buffered saline, the cornea and lens were excised under a microscope, and the retina was separated from the pigment epithelium to completely detach the retina from the sclera. After that, the retina was cut into four quadrants and mounted with Fluoromount (Diagnostic BioSystems, Pleasanton, CA, USA) in a flat state to generate a retinal flat mount specimen.

The retinal flat mount specimen was photographed under a fluorescence microscope (BX50, Olympus) using a high-sensitivity cooling CCD camera (DP30BW, Olympus) through imaging software Metamorph (Universal Imaging Corp., Downingtown, PA, USA) on an XY motor-operated stage (Sigma Koki Co., Ltd., Tokyo, Japan). An image of the whole retina was produced from 13 images. The images were continuously photographed from the surface layer to the lower layer of the retinal blood vessels at intervals of 14.2 µm. The ratio of the retinal non perfusion area was quantified using analysis software in Metamorph. All analyses were performed in a blinded manner. The results of the early administration are shown in FIG. 6(a), and the results of the late administration are shown in FIG. 6(b). The vertical axis represents the ratio of the retinal non perfusion area, and the horizontal axis represents the number of days after occlusion (Time after occlusions (days)).

As shown in FIG. 6(a) and FIG. 6(b), Compound 1 significantly reduced the enlargement of the non perfusion area in both the early administration and the late administration. This shows that Compound 1 has an ischemia-ameliorating action irrespective of whether the disease state of the RVO model is early or late.

### (Example 5) Permeability-reducing Action of Compound 1 on Human Retinal Vascular Endothelial Cells

The upper chamber of Transwell (diameter: 6.5 mm, pore size: 0.4 µm: Corning Incorporated) coated with collagen and fibronectin was seeded with human retinal vascular endothelial cells (HRMECs) (Cell Systems) at a cell density of 5×10⁴ cells/well, and the cells were cultured in Endothelial Cell Growth Medium MV 2 (PromoCell GmbH) under a 5% CO₂ atmosphere at 37°C for 6 days. Then, to the upper chamber and lower chamber of the Transwell, Compound 1 at a final concentration of 0 nM, 0.3 nM, 3 nM, or 30 nM, which had been prepared using 0.001% dimethyl sulfoxide-containing Endothelial Cell Growth Medium MV 2 as a vehicle, was added, and culture was continued. A well to which only the vehicle was added was used as a control. After 60 minutes of culture, 1 mg/mL of fluorescein isothiocyanate (FITC)-dextran (40 kDa: Sigma-Aldrich) was added to the upper chamber of the Transwell, and 60 minutes later, the fluorescence intensity of the medium in the lower chamber was measured to measure the amount of FITC-dextran permeating the lower chamber. The results are shown in FIG. 7. Compound 1 significantly decreased the amount of FITC-dextran permeating the lower chamber.

This shows that Compound 1 has reduced the permeability between the retinal vascular endothelial cells.

### (Example 6) Permeability-reducing Action of Compound 2 on Human Retinal Vascular Endothelial Cells

The permeability-reducing action of Compound 2 on human retinal vascular endothelial cells was measured in the same manner as in Example 5. The results are shown in FIG. 8. Compound 2 significantly decreased the amount of FITC-dextran permeating the lower chamber.

This shows that Compound 2 has reduced the permeability between the retinal vascular endothelial cells.

### (Example 7) Permeability-reducing Action of Compound 3 on Human Retinal Vascular Endothelial Cells

The permeability-reducing action of Compound 3 on human retinal vascular endothelial cells was measured in the same manner as in Example 5. The results are shown in FIG. 9. Compound 3 significantly decreased the amount of FITC-dextran permeating the lower chamber.

This shows that Compound 3 has reduced the permeability between the retinal vascular endothelial cells.

### (Example 8) Permeability-reducing Action of Compound 4 on Human Retinal Vascular Endothelial Cells

The permeability-reducing action of Compound 4 on human retinal vascular endothelial cells was measured in the same manner as in Example 5. The results are shown in FIG. 10. Compound 4 significantly decreased the amount of FITC-dextran permeating the lower chamber.

This shows that Compound 4 has reduced the permeability between the retinal vascular endothelial cells.

### (Example 9) Permeability-reducing Action of Compound 5 on Human Retinal Vascular Endothelial Cells

The permeability-reducing action of Compound 5 on human retinal vascular endothelial cells was measured in the same manner as in Example 5. The results are shown in FIG. 11. Compound 5 significantly decreased the amount of FITC-dextran permeating the lower chamber.

This shows that Compound 5 has reduced the permeability between the retinal vascular endothelial cells.

### (Example 10) Permeability-reducing Action of Compound 6 on Human Retinal Vascular Endothelial Cells

The permeability-reducing action of Compound 6 on human retinal vascular endothelial cells was measured in the same manner as in Example 5. The results are shown in FIG. 12. Compound 6 significantly decreased the amount of FITC-dextran permeating the lower chamber.

This shows that Compound 6 has reduced the permeability between the retinal vascular endothelial cells.

### (Example 11) Permeability-reducing Action of Compound 7 on Human Retinal Vascular Endothelial Cells

The permeability-reducing action of Compound 7 on human retinal vascular endothelial cells was measured in the same manner as in Example 5. The results are shown in FIG. 13. Compound 7 significantly decreased the amount of FITC-dextran permeating the lower chamber.

This shows that Compound 7 has reduced the permeability between the retinal vascular endothelial cells.

### (Comparative Example 1) Effects of FTY720 on CNV Model (1) Evaluation of Fluorescein Angiography (FA) Grade

A Mydrin-P ophthalmic solution (Santen Pharmaceutical Co., Ltd., Mydrin is a trademark) was dropped into one eye of 8-week-old mice (Japan SLC, Inc., C56BL/6J strain) to cause mydriasis, and then six points on the periphery of the optic nerve head at equal intervals were subjected to laser irradiation by the same method as in Example 1.

FTY720 was suspended in a vehicle (0.5% MC solution), and was orally administered at a dose of 0.3 mg/kg once daily from the day before the laser irradiation to the day before sampling. A control was administered only the vehicle (vehicle group) . Experiments were performed using 16 mice in each of the FTY720 administration group and the vehicle group.

Sampling was performed on the 14th day after the laser irradiation, and then fluorescence fundus photography was performed by the same method as in Example 1.

The results are shown in FIG. 14(a). In FIG. 14(a), Day 0 indicates images taken on the day of the laser irradiation, and Day 14 indicates images taken on the 14th day. In addition, for the image of each group on Day 14, evaluation was performed by the same method as in Example 1. The results are shown in FIG. 14(b).

In the group administered FTY720 serving as a non-selective S1P receptor agonist, a significant lowering in FA grade was not found as compared to the vehicle group.

### (2) Evaluation of Area of CNV

After fluorescence fundus photography had been performed as described above in the section (1), a choroidal flat mount specimen was generated by the same method as in Example 1. The choroidal flat mount was photographed using an all-in-one fluorescence microscope (BZ-X710; Keyence, Osaka, Japan). The resultant images are shown in FIG. 15(a).

Next, on the basis of the photographed image, the CNV was encircled using image editing software Image J, and the area thereof was determined as the area of CNV (µm²). All analyses were performed in a blinded manner. The results are shown in FIG. 15(b).

The administration of FTY720 was not found to significantly decrease the area of CNV indicating new blood vessels.

The above-mentioned results confirmed that FTY720 serving as a non-selective S1P receptor agonist did not reduce neovascularization in this model.

In Comparative Example 1, a significant change in body weight between the groups before sampling was not found. A case in which FA was unable to be performed owing to cloudiness caused by a flaw in the eye produced before the FA evaluation and a case in which CNV was connected at the time of the evaluation of the area of CNV were excluded from analysis, and the above-mentioned results were each calculated as a mean of 12 mice in each group.

### (Example 12) Effect by Intravitreal Administration in RVO Model

Eight-week-old mice (Japan SLC, Inc., ddy strain) were anesthetized by intramuscularly administering 10 mL/kg of a mixed anesthetic solution diluted so as to contain ketamine (120 mg/kg) and xylazine (6 mg/kg). After that, 0.15 mL of rose bengal (20 mg/kg; WAKO) was administered into the tail vein, and a vein three head systems away from the optic nerve head of the right eye of the mice was irradiated with a laser to occlude a retinal vein. For the occlusion, the laser irradiation was performed 10 times to 15 times per vein. For the laser irradiation, an attachment of a fundus photography apparatus Micron 4 (Phoenix Research Laboratories, Inc., Pleasanton, CA, USA) was used (wavelength: 532 nm, spot size: 50 µm, irradiation time: 5,000 ms, laser output: 50 mW). Three veins were occluded per eye, and it was confirmed with photographs of the fundus that the laser-irradiated veins had been completely occluded.

Compound 1 was dissolved in a vehicle (4 wt% polyethylene glycol 400, 0.1 wt% polysorbate 80, 0.01 wt% dimethyl sulfoxide-containing phosphate-buffered saline), and was intravitreally administered to the right eye immediately after the laser irradiation at a dose of 0.8 ng, 8 ng, or 80 ng of Compound 1 (in terms of free form) per eye. A control was administered only the vehicle (vehicle group). In addition, the left eye (not laser-irradiated and not administered any substance) of mice whose right eye was administered only the vehicle was used as a normal group. Experiments were performed using 6 mice in each of the Compound 1 administration groups, the normal group, and the vehicle group.

After 1 day from the laser irradiation, the mice were euthanized by cervical dislocation, the eyeball was extirpated, and histological evaluation was performed by a hematoxylin-eosin staining method. In addition, the thickness of the retinal inner nuclear layer (INL) was measured at intervals of 240 µm on both sides, i.e., ear side and nose side from the center of the optic nerve head. All analyses were performed in a blinded manner. Stained images in the histological evaluation are shown in FIG. 16(a), the measurement results of the INL thickness are shown in FIG. 16(b), and the mean of the thicknesses of the INL is shown in FIG. 16(c).

As shown in FIG. 16(a) to FIG. 16(c), it was revealed that Compound 1 ameliorated the thickening of the INL and ameliorated retinal edema also through intravitreal administration.

### Industrial Applicability

The selective S1P receptor agonist having agonist activity at the S1P₁ receptor described in the present application can be used as a preventive or therapeutic agent for an ophthalmic disease associated with intraocular neovascularization and/or increased intraocular vascular permeability, specifically, for example, exudative age-related macular degeneration, diabetic retinopathy, diabetic macular edema, retinopathy of prematurity, myopic choroidal neovascularization, secondary choroidal neovascularization, retinal artery occlusion, retinal vein occlusion, neovascular glaucoma, retinitis pigmentosa, or edema caused by retinal photocoagulation.

## Claims

1. A pharmaceutical composition for preventing or treating an ophthalmic disease associated with intraocular neovascularization and/or increased intraocular vascular permeability, the pharmaceutical composition comprising as an active ingredient a selective S1P receptor agonist having agonist activity at an S1P₁ receptor.

2. The pharmaceutical composition according to claim 1, wherein the selective S1P receptor agonist is a compound having S1P₁ receptor agonist activity and further having agonist activity at one or both of an S1P₅ receptor and an S1P₄ receptor, or a pharmaceutically acceptable salt thereof.

3. The pharmaceutical composition according to claim 1, wherein the selective S1P receptor agonist is a compound having S1P₁ receptor agonist activity and further having agonist activity at an S1P₅ receptor, or a pharmaceutically acceptable salt thereof.

4. The pharmaceutical composition according to any one of claims 1 to 3, wherein the selective S1P receptor agonist is a compound or a pharmaceutically acceptable salt thereof selected from the group consisting of: 5-{5-[3-(trifluoromethyl)-4-{[(2S)-1,1,1-trifluoropropan-2-yl] oxy}phenyl]-1,2,4-oxadiazol-3-yl}-1H-benzimidazole or a pharmaceutically acceptable salt thereof; 1-[(7-{[4-(2,2,2-trifluoroethoxy)-3-(trifluoromethyl)phenyl]me thoxy}-2H-1-benzopyran-3-yl)methyl]piperidine-4-carboxylic acid or a pharmaceutically acceptable salt thereof; 1-({4-[(1E)-N-{[4-cyclohexyl-3-(trifluoromethyl)phenyl]methoxy }ethanimidoyl]-2-ethylphenyl}methyl)azetidine-3-carboxylic acid or a pharmaceutically acceptable salt thereof; 5-(3-{(1S)-1-[(2-hydroxyethyl)amino]-2,3-dihydro-1H-inden-4-yl }-1,2,4-oxadiazol-5-yl)-2-[(propan-2-yl)oxy]benzonitrile or a pharmaceutically acceptable salt thereof; (2Z,5Z)-5-({3-chloro-4-[(2R)-2,3-dihydroxypropoxy]phenyl}methy lidene)-3-(2-methylphenyl)-2-(propylimino)-1,3-thiazolidin-4-o ne or a pharmaceutically acceptable salt thereof; [(3R)-7-{[4-cyclopentyl-3-(trifluoromethyl)phenyl]methoxy}-1,2 ,3,4-tetrahydrocyclopenta[b]indol-3-yl]acetic acid or a pharmaceutically acceptable salt thereof; and 2-amino-2-[2-(4-{[3-(benzyloxy)phenyl]sulfanyl}-2-chlorophenyl )ethyl]propane-1,3-diol or a pharmaceutically acceptable salt thereof.

5. The pharmaceutical composition according to claim 4, wherein the selective S1P receptor agonist is 5-{5-[3-(trifluoromethyl)-4-{[(2S)-1,1,1-trifluoropropan-2-yl] oxy}phenyl]-1,2,4-oxadiazol-3-yl}-1H-benzimidazole or a pharmaceutically acceptable salt thereof.

6. The pharmaceutical composition according to any one of claims 1 to 5, wherein the ophthalmic disease associated with intraocular neovascularization and/or increased intraocular vascular permeability is exudative age-related macular degeneration, diabetic retinopathy, diabetic macular edema, retinopathy of prematurity, myopic choroidal neovascularization, secondary choroidal neovascularization, retinal artery occlusion, retinal vein occlusion, neovascularglaucoma, retinitispigmentosa, or edema caused by retinal photocoagulation.

7. The pharmaceutical composition according to any one of claims 1 to 5, wherein the ophthalmic disease associated with intraocular neovascularization and/or increased intraocular vascular permeability is exudative age-related macular degeneration, diabetic retinopathy, diabetic macular edema, myopic choroidal neovascularization, retinal artery occlusion, retinal vein occlusion, or neovascular glaucoma.

8. The pharmaceutical composition according to any one of claims 1 to 7, wherein the pharmaceutical composition is intraocularly injected or intravitreally injected to treat the ophthalmic disease associated with intraocular neovascularization and/or increased intraocular vascular permeability.

9. The pharmaceutical composition according to claim 8, wherein the pharmaceutical composition is intravitreally injected to treat the ophthalmic disease associated with intraocular neovascularization and/or increased intraocular vascular permeability.

10. A method of preventing or treating an ophthalmic disease associated with intraocular neovascularization and/or increased intraocular vascular permeability, the method comprising administering an effective dose of a selective S1P receptor agonist having agonist activity at an S1P₁ receptor to a subject.

11. The method according to claim 10, wherein the selective S1P receptor agonist is a compound having S1P₁ receptor agonist activity and further having agonist activity at one or both of an S1P₅ receptor and an S1P₄ receptor, or a pharmaceutically acceptable salt thereof.

12. The method according to claim 10, wherein the selective S1P receptor agonist is a compound having S1P₁ receptor agonist activity and further having agonist activity at an S1P₅ receptor, or a pharmaceutically acceptable salt thereof.

13. The method according to any one of claims 10 to 12, wherein the selective S1P receptor agonist is a compound or a pharmaceutically acceptable salt thereof selected from the group consisting of: 5-{5-[3-(trifluoromethyl)-4-{[(2S)-1,1,1-trifluoropropan-2-yl] oxy}phenyl]-1,2,4-oxadiazol-3-yl}-1H-benzimidazole or a pharmaceutically acceptable salt thereof; 1-[(7-{[4-(2,2,2-trifluoroethoxy)-3-(trifluoromethyl)phenyl]me thoxy}-2H-1-benzopyran-3-yl)methyl]piperidine-4-carboxylic acid or a pharmaceutically acceptable salt thereof; 1-({4-[(1E)-N-{[4-cyclohexyl-3-(trifluoromethyl)phenyl]methoxy }ethanimidoyl]-2-ethylphenyl}methyl)azetidine-3-carboxylic acid or a pharmaceutically acceptable salt thereof; 5-(3-{(1S)-1-[(2-hydroxyethyl)amino]-2,3-dihydro-1H-inden-4-yl }-1,2,4-oxadiazol-5-yl)-2-[(propan-2-yl)oxy]benzonitrile or a pharmaceutically acceptable salt thereof; (2Z,5Z)-5-({3-chloro-4-[(2R)-2,3-dihydroxypropoxy]phenyl}methy lidene)-3-(2-methylphenyl)-2-(propylimino)-1,3-thiazolidin-4-o ne or a pharmaceutically acceptable salt thereof; [(3R)-7-{[4-cyclopentyl-3-(trifluoromethyl)phenyl]methoxy}-1,2 ,3,4-tetrahydrocyclopenta[b]indol-3-yl]acetic acid or a pharmaceutically acceptable salt thereof; and 2-amino-2-[2-(4-{[3-(benzyloxy)phenyl]sulfanyl}-2-chlorophenyl )ethyl]propane-1,3-diol or a pharmaceutically acceptable salt thereof.

14. The method according to claim 13, wherein the selective S1P receptor agonist is 5-{5-[3-(trifluoromethyl)-4-{[(2S)-1,1,1-trifluoropropan-2-yl] oxy}phenyl]-1,2,4-oxadiazol-3-yl}-1H-benzimidazole or a pharmaceutically acceptable salt thereof.

15. The method according to any one of claims 10 to 14, wherein the ophthalmic disease associated with intraocular neovascularization and/or increased intraocular vascular permeability is exudative age-related macular degeneration, diabetic retinopathy, diabetic macular edema, retinopathy of prematurity, myopic choroidal neovascularization, secondary choroidal neovascularization, retinal artery occlusion, retinal vein occlusion, neovascularglaucoma, retinitis pigmentosa, or edema caused by retinal photocoagulation.

16. The method according to any one of claims 10 to 14, wherein the ophthalmic disease associated with intraocular neovascularization and/or increased intraocular vascular permeability is exudative age-related macular degeneration, diabetic retinopathy, diabetic macular edema, myopic choroidal neovascularization, retinal artery occlusion, retinal vein occlusion, or neovascular glaucoma.

17. The method according to any one of claims 10 to 16, wherein a method for the administering is intraocular injection or intravitreal injection.

18. The method according to claim 17, wherein the method for the administering is intravitreal injection.

19. A selective S1P receptor agonist having agonist activity at an S1P₁ receptor, for use in prevention or treatment of an ophthalmic disease associated with intraocular neovascularization and/or increased intraocular vascular permeability.

20. The selective S1P receptor agonist according to claim 19, wherein the selective S1P receptor agonist is a compound having S1P₁ receptor agonist activity and further having agonist activity at one or both of an S1P₅ receptor and an S1P₄ receptor, or a pharmaceutically acceptable salt thereof.

21. The selective S1P receptor agonist according to claim 19, wherein the selective S1P receptor agonist is a compound having S1P₁ receptor agonist activity and further having agonist activity at an S1P₅ receptor, or a pharmaceutically acceptable salt thereof.

22. The selective S1P receptor agonist according to any one of claims 19 to 21, wherein the selective S1P receptor agonist is a compound or a pharmaceutically acceptable salt thereof selected from the group consisting of: 5-{5-[3-(trifluoromethyl)-4-{[(2S)-1,1,1-trifluoropropan-2-yl] oxy}phenyl]-1,2,4-oxadiazol-3-yl}-1H-benzimidazole or a pharmaceutically acceptable salt thereof; 1-[(7-{[4-(2,2,2-trifluoroethoxy)-3-(trifluoromethyl)phenyl]me thoxy}-2H-1-benzopyran-3-yl)methyl]piperidine-4-carboxylic acid or a pharmaceutically acceptable salt thereof; 1-({4-[(1E)-N-{[4-cyclohexyl-3-(trifluoromethyl)phenyl]methoxy }ethanimidoyl]-2-ethylphenyl}methyl)azetidine-3-carboxylic acid or a pharmaceutically acceptable salt thereof; 5-(3-{(1S)-1-[(2-hydroxyethyl)amino]-2,3-dihydro-1H-inden-4-yl }-1,2,4-oxadiazol-5-yl)-2-[(propan-2-yl)oxy]benzonitrile or a pharmaceutically acceptable salt thereof; (2Z,5Z)-5-({3-chloro-4-[(2R)-2,3-dihydroxypropoxy]phenyl}methy lidene)-3-(2-methylphenyl)-2-(propylimino)-1,3-thiazolidin-4-o ne or a pharmaceutically acceptable salt thereof; [(3R)-7-{[4-cyclopentyl-3-(trifluoromethyl)phenyl]methoxy}-1,2 ,3,4-tetrahydrocyclopenta[b]indol-3-yl]acetic acid or a pharmaceutically acceptable salt thereof; and 2-amino-2-[2-(4-{[3-(benzyloxy)phenyl]sulfanyl}-2-chlorophenyl )ethyl]propane-1,3-diol or a pharmaceutically acceptable salt thereof.

23. The selective S1P receptor agonist according to claim 22, wherein the selective S1P receptor agonist is 5-{5-[3-(trifluoromethyl)-4-{[(2S)-1,1,1-trifluoropropan-2-yl] oxy}phenyl]-1,2,4-oxadiazol-3-yl}-1H-benzimidazole or a pharmaceutically acceptable salt thereof.

24. The selective S1P receptor agonist according to any one of claims 19 to 23, wherein the ophthalmic disease associated with intraocular neovascularization and/or increased intraocular vascular permeability is exudative age-related macular degeneration, diabetic retinopathy, diabetic macular edema, retinopathy of prematurity, myopic choroidal neovascularization, secondary choroidal neovascularization, retinal artery occlusion, retinal vein occlusion, neovascularglaucoma, retinitispigmentosa, or edema caused by retinal photocoagulation.

25. The selective S1P receptor agonist according to any one of claims 19 to 23, wherein the ophthalmic disease associated with intraocular neovascularization and/or increased intraocular vascular permeability is exudative age-related macular degeneration, diabetic retinopathy, diabetic macular edema, myopic choroidal neovascularization, retinal artery occlusion, retinal vein occlusion, or neovascular glaucoma.

26. The selective S1P receptor agonist according to any one of claims 19 to 25, wherein the selective S1P receptor agonist is for use in intraocular injection or intravitreal injection.

27. The selective S1P receptor agonist according to claim 26, wherein the selective S1P receptor agonist is for use in intravitreal injection.
